# EUROPEAN PATENT APPLICATION

(11) **EP 3 967 314 A1**
(43) Date of publication of application: **16.03.2022**
(21) Application number: 20306033.0
(22) Date of filing: 15.09.2020
(51) Int. Cl.: A61K 35/741, A23L 33/135, A61P 1/00, A61P 3/04, A61P 29/00

(54) **PARABACTEROIDES DISTASONIS STRAINS FOR USE THEREOF IN THE TREATMENT AND PREVENTION OF GASTROINTESTINAL DISEASES AND OF DISORDERS ASSOCIATED WITH GASTROINTESTINAL DISEASES**

(71) Applicant: Institut national de recherche pour l'agriculture l'alimentation et l'environnement, 75007 Paris (FR); Université de Paris, 75006 Paris (FR); Institut Pasteur de Lille, 59000 Lille (FR); INSERM (Institut National de la Santé et de la Recherche Médicale), 75013 Paris Cédex 13 (FR); Centre national de la recherche scientifique, 75016 Paris (FR); Université de Lille, 59000 Lille (FR)
(72) Inventor: MAGUIN, Emmanuelle, 91440 Bures-sur-Yvette (FR); GRANGETTE, Corinne, 59650 Villeneuve d'Ascq (FR); CUFFARO, Bernardo, 59370 Mons-en-Baroeul (FR); WALIGORA-DUPRIET, Anne-Judith, 92140 Clamart (FR)
(74) Representative: Nony

(57) **Abstract**

The present invention relates to a bacterial strain of the species *Parabacteroides distasonis* for use thereof in the treatment and/or prevention of a gastrointestinal disease, or of a disorder associated with gastrointestinal disease, in an individual,
said bacterial strain being selected from the group consisting of:
- the bacterial strain deposited with the CNCM under accession number CNCM 1-5576; and
- the bacterial strain deposited with the CNCM under accession number CNCM 1-5578.

It further relates to a bacterial strain deposited with the CNCM under accession number CNCM 1-5576 and to a bacterial strain deposited with the CNCM under accession number CNCM 1-5578.

The present invention finally relates to a composition comprising, in a physiologically acceptable medium, a least a bacterial strain of the invention.

## Description

### Field of the invention

The present invention relates to *Parabacteroides distasonis* strains for use thereof in the treatment and prevention of gastrointestinal diseases in an individual as well as in the treatment and prevention of disorders associated with gastrointestinal diseases, such as obesity.

More particularly, the present invention relates to *Parabacteroides distasonis* strains for use thereof in the treatment and prevention of:
- inflammatory gastrointestinal diseases;
- disorders associated with an inflammatory gastrointestinal disease; and/or
- a leaky gastrointestinal mucosal barrier.

### Prior art

Inflammation is a natural biological process which constitutes a normal part of the response to lesions or infections and contributes to protecting the organism against internal or external attacks.

However, a dysfunction of the inflammation mechanisms, in particular a persistent or excessively abundant inflammation, may cause painful diseases and endanger the patient's life. Such diseases comprise, for example, skin disorders, bowel disorders, neurological disorders, arthritis and autoimmune diseases. Several of these inflammatory diseases still have no treatment or have no appropriate treatment.

Consequently, the study of and search for new anti-inflammatory treatment strategies constitutes a major subject in medicine and in biomedical research.

An inflammatory bowel disease is a group of disorders characterized by a chronic and recurrent inflammation of the gastrointestinal tract. The most common form of this group is Crohn's disease. The pathogenesis involves inappropriate and continuous activation of the mucosal immune system caused by the presence of the intestinal microbiota in a genetically predisposed patient.

Another factor playing a role in the appearance of inflammatory disorders in the gastrointestinal tract is the alteration of the intestinal permeability.

The gastroinstestinal tract is indeed in particular characterized in that it comprises a contiguous layer of cells whose function is to separate the external environment, i.e. the lumen of the bowel, from the inside of the body. This function is highly important because of the numerous environmental agents that can be harmful to the organism if they cross the epithelial barrier. The intestine of course normally exhibits some permeability to allow nutrients and water to pass through the epithelial barrier and does not usually let particles bigger than about 15 Å pass into the organism.

However, for various reasons, the permeability of the epithelial barrier can increase, notably through the disruption of tight junction proteins, which allows many elements from the lumen to bypass the epithelial barrier and to penetrate the intestinal wall and thus the organism. Experiments performed on rats in the 1990s, where luminal compounds were directly placed into the colonic wall of said rats initiated inflammatory diseases. Accordingly, maintaining or restauring the epithelial barrier function in the gastrointestinal tract of an individual can significantly help preventing the onset of a gastrointestinal inflammation.

Moreover, an increasing number of studies suggest that a reduction of the gut barrier function, which can also be called intestinal hyperpermeability or leaky gut or leaky gastrointestinal mucosal barrier, can play an important role in the onset of various diseases in many parts of the body, such as for example obesity and diabetes. Indeed, studies have demonstrated that not only people suffering from diabetes, in particular from type 1 diabetes, but also pre-diabetic individuals, present an increased permeability.

Accordingly, preventing and/or treating a leaky gut is not only a good way of preventing the onset of gastrointestinal inflammatory disorders, but also of non-inflammatory gastrointestinal disorders such as IBS (Irritable Bowel Syndrome) and of non-gastrointestinal disorders.

There is a constant need for novel substances, in particular probiotics and biotherapeutic products, or compositions for the treatment and/or prevention of gastrointestinal diseases, inparticular inflammatory gastrointestinal diseases, and more particularly inflammatory bowel diseases, in an individual.

The mammalian gut harbors a complex and dynamic community of microorganisms, collectively called the gut microbiota, including archeae, fungi, viruses, protists, helminths and mainly bacteria (Hoffmann, C. et al. PLoS ONE 2013, 8, e66019 and Qin, J. et al. Nature 2010, 464, 59-65). Among bacteria, *Bacteroidetes* and *Firmicutes* are the most abundant phyla together with less abundant *Proteobacteria, Actinobacteria, Fusobacteria* and *Verrucomicrobia.* Despite a high inter-individual variability, metagenomic analysis highlighted a core functional gut microbiome including approximately 60 bacterial species shared by healthy subjects (Human Microbiome Project Consortium Structure, function and diversity of the healthy human microbiome. Nature 2012, 486, 207-214 and Turnbaugh, P.J.; Gordon, J.I. J., Physiol. (Lond.) 2009, 587, 4153-4158). This microbial community lives in a symbiotic relationship with the host displaying important metabolic, immunologic and gut protective functions (Bäckhed, F. et al. Science 2005, 307, 1915-1920 and Slack, E. et al. Science 2009, 325, 617-620). It notably exerts a pivotal role in educating and orchestrating both the innate and adaptive immune response at the intestinal level. Indeed, the microbiota plays a key role in maintaining the intestinal epithelial barrier function, the immune homeostasis and the protection against pathogen colonization (Pickard, J.M. et al. Immunol. Rev. 2017, 279, 70-89).

Alterations in microbiota composition together with reduced bacterial diversity, known as dysbiosis, are frequently observed in patients suffering from a variety of chronic disorders including inflammatory bowel disease (IBD) (Frank, D.N. et al. Proc. Natl. Acad. Sci. U.S.A. 2007, 104, 13780-13785, Gevers, D. et al. Cell Host Microbe 2014, 15, 382-392 and Manichanh, C. et al. Gut 2006, 55, 205-211), obesity (Le Chatelier, E. et al. Nature 2013, 500, 541-546; Ley, R.E. et al. Proc. Natl. Acad. Sci. U.S.A. 2005, 102, 11070-11075, and Turnbaugh, P.J. et al. Nature 2006, 444, 1027-1031), type 1 and 2 diabetes (Kostic, A.D. et al. Cell Host Microbe 2015, 17, 260-273 and Qin, J. et al. Nature 2012, 490, 55-60). However, it remains difficult to establish a definitive causal role of the gut microbiota to such diseases. The role of bacteria in IBD has been strongly supported in animal models in which no colitis occurred after eradication of the colonic microbiota by antibiotics or the use of germ-free animals (Sartor, R.B. Gastroenterology 2004, 126, 1620-1633 and Sellon, R.K. et al. Infect. Immun. 1998, 66, 5224-5231). Moreover, microbiota transplantation from IBD patients or colitic animals to axenic recipient mice led to gut inflammation arguing that dysbiotic microbiota play an important role in the pathogenesis of inflammatory disease (Butto, L.F.; Haller, D. International Journal of Medical Microbiology 2016, 306, 302-309 and Schaubeck, M. et al. Gut 2016, 65, 225-237).

Despite fast gathering of omics data documenting the change of the intestinal microbiota in IBD, consensus about specific disease-relevant taxa remains weak, even if dysbiosis has been linked with the expansion of some pathobionts notably belonging to *Enterobacteriaceae* in about 30% of Crohn patients (Baumgart, M. et al. ISME J 2007, 1, 403-418 and Darfeuille-Michaud, A. Int. J. Med. Microbiol. 2002, 292, 185-193) and some bacterial (Pascal et al. Gut, 2017, doi n° 10.1136/gutjnl-2016-313235) and fungal signatures (Sokol et al. Gut, 2017 doi n° 10.1136/gutjnl-2015-310746). Of note, *Parabacteroides* belong to the 26 bacterial genus significantly enriched in the microbiome of healthy controls compare with Crohn patients (Pascal et al. Gut, 2017 Gut, doi n° 10.1136/gutjnl-2016-313235). Conversely, IBD is characterized by the alteration in *Firmicutes* abundance, especially reduction in *Clostridium* cluster XIVa and IV, with *Faecalibacterium prausnitzii* as the dominant species (Frank, D.N. et al. Proc. Natl. Acad. Sci. U.S.A. 2007, 104, 13780-13785; Lopez-Siles, M. et al. Appl. Environ. Microbiol. 2015, 81, 7582-7592 and Sokol, H. et al. Inflamm. Bowel Dis. 2009, 15, 1183-1189). The therapeutic strategies for patients with IBD, mainly based the use of anti-inflammatory and/or immunosuppressive drugs are not curative and in addition, about 25-30% of patients fail to respond and 20% of the patients will discontinue therapy due to side-effects (Dave, M. et al. Inflamm. Bowel Dis. 2014, 20, 196-212 and Chan, H.C.; Ng, S.C. J Gastroenterol 2017, 52, 141-150).

Therefore, targeting the microbiota dysbiosis is becoming a forefront of biomedical research. Fecal microbial transplantation (FMT) has emerged as a putative microbiome-targeting therapy in IBD (Fang, H. et al. Biomed Res Int 2018, 2018), however, determining the best donor and standardization of the protocol is still a challenge (El Hage, R. et al. Frontiers in Microbiology 2017, 8) and the success rate remains limited today (Kong et al. 2020).

In this context, the use of probiotics has received a lot of attention in the last decade because of the "natural" and safety aspects of such treatment (Parvez, S. et al. J. Appl. Microbiol. 2006, 100, 1171-1185). Traditional probiotics are indeed based to their classification as QPS (Qualified Presumption of Safety) by the European Food Safety Authority (EFSA) or GRAS (Generally Regarded as Safe) by the United States Food and Drug Administration (FDA). They mostly include limited number of genera, mainly belonging to *Lactobacillus spp.* and *Bifidobacterium spp.* with a long history of use with proved biological safety. Many beneficial effects have been attributed to such microorganisms using various *in vitro* and animal experimental models. The inventors and others have reported beneficial impact of traditional probiotics administration in mice, highlighting their capacity to promote immune regulatory responses (Foligne, B. et al. PLoS ONE 2007, 2, e313 and Macho Fernandez, E. et al. Gut 2011, 60, 1050-1059), antimicrobial responses (Hrdy et al, Sci. Rep. 2020, 10, 5345) and epithelial barrier strengthening (Alard, J. et al. Benef Microbes 2018, 9, 317-331 and Zaylaa, M. et al. Journal of Functional Foods 2018, 47, 304-315). Systematic review of randomized controlled trials reported some efficacy of probiotics in induction or maintenance of remission in ulcerative colitis and pouchitis, however data are insufficient to recommend probiotics for use in Crohn's disease (Ghouri, Y.A. et al. Clin Exp Gastroenterol 2014, 7, 473-487 and Saez-Lara, M.J. et al. Biomed Res Int 2015, 2015, 505878).

Based on this low effectiveness and considering the increased knowledge about the gut microbiota, identification and characterization of novel and disease-specific health-promoting bacteria commonly called next generation probiotics (NGP) start to emerge as new preventive and therapeutic tools. The beneficial effects of *F. prausnitzii* as a NGP have been successfully shown in several murine models of IBD and IBS (Sokol, H. et al. Proc. Natl. Acad. Sci. U.S.A. 2008, 105, 16731-16736) and is also currently under clinical investigation. These results provided a rationale for the use of bacteria isolated from the gut microbiota as a source of NGP.

There is thus a need for novel substances, and in particular next generation probiotics, for the prevention or treatment of diseases associated with microbiota dysbiosis, in particular of gastrointestinal diseases and disorders associated with gastrointestinal diseases, more particularly of inflammatory gastrointestinal diseases or of a disorder associated with gastrointestinal inflammation such as obesity.

There is also a need for novel substances, and in particular next generation probiotics, for the prevention or treatment of a leaky gastrointestinal mucosal barrier, also termed a leaky gut.

### Summary of the invention

The objective of the present invention is to provide novel substances, in particular next generation probiotics (NGP) or pharmabiotics (Shanahan et al. Funct Food Rev. 2009;1:20-25), and compositions comprising them, for the treatment and/or prevention of gastrointestinal diseases, as well as in the treatment and/or prevention of disorders associated with a gastrointestinal disease, and more particularly for the treatment and/or prevention of (i) inflammatory gastrointestinal diseases; (ii) disorders associated with an inflammatory gastrointestinal disease; and/or (iii) a leaky gastrointestinal mucosal barrier.

According to a first subject, the present invention relates to a bacterial strain of the species *Parabacteroides distasonis* for use thereof in the treatment and/or prevention of a gastrointestinal disease, or of a disorder associated with a gastrointestinaldisease, in an individual, said bacterial strain being selected from the group consisting of:
- the bacterial strain deposited with the CNCM under accession number CNCM 1-5576; and
- the bacterial strain deposited with the CNCM under accession number CNCM 1-5578.

In a particular embodiment, the bacterial strain of the species Parabacteroides distasonis is for use in the treatment and/or prevention of:
- inflammatory gastrointestinal diseases;
- disorders associated with an inflammatory gastrointestinal disease; and/or
- a leaky gastrointestinal mucosal barrier.

The inventors have indeed unexpectedly discovered that two bacterial strains from the species *Parabacteroides distasonis* possess various advantageous properties and are in particular able to treat and prevent inflammatory gastrointestinal diseases, disorders associated with an inflammatory gastrointestinal disease and to prevent the alteration of the permeability of the gut barrier and restaure the gut barrier. These bacterial strains are those deposited with the CNCM under accession number CNCM 1-5576; and the bacterial strain deposited with the CNCM under accession number CNCM 1-5578.

According to the experimental results of the inventors, these two specific strains of *Parabacteroides distasonis* (*P. distasonis*) deposited with the CNCM on September 7, 2020, under accession numbers CNCM 1-5576 and CNCM 1-5578 have the unexpected capacities to:
- reduce, *in vitro* and *in vivo,* a gastrointestinal inflammation, in particular a bowel inflammation, in an individual.
- restore the altered permeability of the gut, and thus the barrier function of the gut, in particular when the gut presents a strengthened permeability compared to its permeability when the gut is in an unhealthy or healthy state. Such properties are even demonstrated as being independent from the presence an inflammation.
- limit weight gain and reduce the body weight increase of mice on an obesogenic diet;
- limit the development of fat depots in mice receiving an obesogenic diet; and
- limit the local inflammation in the visceral adipose tissues in mice on an obesogenic diet.

As illustrated in the examples and described hereinafter, the inventors have demonstrated that, on the other hand, the *P. distasonis* strain deposited with the CNCM on September 7, 2020 under accession number CNCM 1-5577 does not possess such advantageous properties, which clearly illustrates the fact that the advantageous properties identified herein cannot be expected from all and any *P. distasonis* bacterial strain.

The strains according to the invention are not only capable of restoring and strengthening the epithelial barrier, and to prevent and treat obesity, but also present strong anti-inflammatory profiles, as they are for example able to increase the production of anti-inflammatory molecules, such as interleukins 6 (IL-6) and 10 (IL-10).

Finally, the inventors were able to demonstrate that the *in vivo* administration of the strains according to the invention protected mice from colitis induced by TNBS.

In a particular embodiment, the bacterial strain of the invention is in a live, semi-active, inactivated, such as inactivated by heat, and/or dead form, and in particular in a live form or in an inactivated by heat form.

An individual according to the invention is preferably a mammal, including a non-human mammal, and is, in particular, a human being.

In a particular embodiment, the individual of the invention is an infant or a child, and in particular is a newborn infant. In a more particular embodiment, the infant is a premature infant and/or an infant born by caesarean section.

In a particular embodiment, the inflammatory gastrointestinal disease is an inflammatory bowel disease (IBD), more particularly a colonic inflammatory bowel disease.

Said colonic inflammatory bowel disease may in particular be chosen from the group consisting of Crohn's disease, ulcerative colitis and pouchitis, in particular the group consisting of Crohn's disease and ulcerative colitis.

In a particular embodiment, the disorder associated with gastrointestinal inflammation is obesity.

According to another embodiment, the bacterial strain for use thereof according to the invention is in a composition comprising a physiologically acceptable medium.

In a particular embodiment, the composition for use according to the invention comprises:
- the bacterial strain deposited with the CNCM under accession number CNCM 1-5576; and
- the bacterial strain deposited with the CNCM under accession number CNCM 1-5578.

In a particular embodiment, a composition of the invention is suitable for the administration of a daily dose representing from 10⁷ to 10¹¹ colony-forming units (cfu) as a medicament, preferably in the form of a daily dose equivalent to 10¹⁰ cfu of the bacterial strain(s).

A composition of the invention is in particular for oral administration.

A composition of the invention for oral administration may be chosen from the group consisting of a food product, a beverage, a pharmaceutical product, a nutraceutical, a food additive, a food supplement and a milk product and is, in particular, in the form of a food supplement.

A composition of the invention may further comprise at least one from the list of ingredients consisting of antioxidants, fish oils, DHA, EPA, vitamins, minerals, phytonutrients, a protein, a lipid, probiotics, prebiotics, fibers, postbiotics, polyphenols, flavonoids, and combinations thereof.

According to another object, the present invention also relates to a bacterial strain deposited with the CNCM under accession number CNCM 1-5576 .

According to another object, the present invention also relates to a bacterial strain deposited with the CNCM under accession number CNCM 1-5578.

A further object of the invention relates to a composition comprising, in a physiologically acceptable medium, at least a bacterial strain of the invention, i.e. a bacterial strain deposited with the CNCM under accession number CNCM 1-5576 and/or a bacterial strain deposited with the CNCM under accession number CNCM 1-5578.

### Figure legends

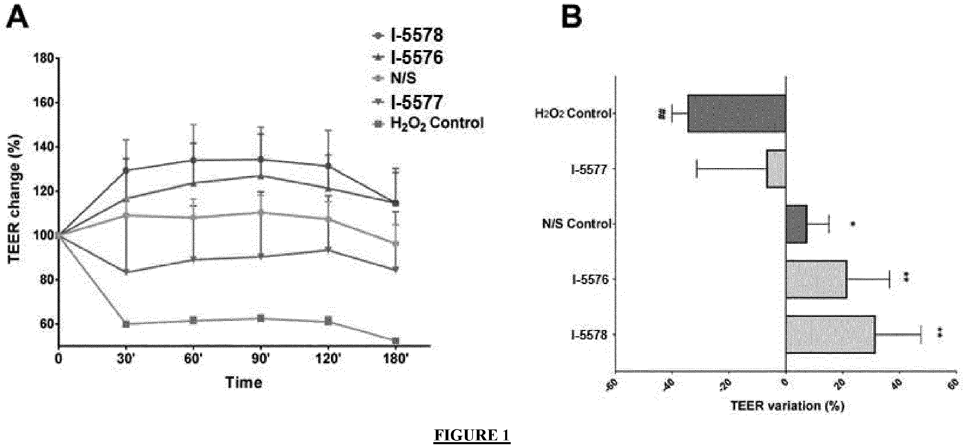

Figure 1 illustrates the ability of the tested strains to restore the H₂O₂-induced disruption of the epithelial barrier. The Caco-2 confluent monolayer cells were pre-treated with the bacteria at the apical side (10:1 bacteria/cell ratio) for 30 min and sensitized with H₂O₂ (100 µM). TEER (Transepithelial electrical resistance) was measured at To and every 30 min. Data represent **(A)** the time-dependent means of relative changes (in %) of TEER ± SEM in comparison to TEER at To and **(B)** the final % TEER variations at 120 min. * refers to the comparison of the Caco-2 cells treated with H₂O₂ and bacteria (CNCM 1-5577 strain outside the invention or CNCM I-5576and CNCM 1-5578 strains according to the invention) or Caco-2 cells without H₂O₂ (N/S Control) versus the Caco-2 cells sensitized with H₂O₂ (H₂O₂ Control); * p < 0.05; ** p < 0.01.

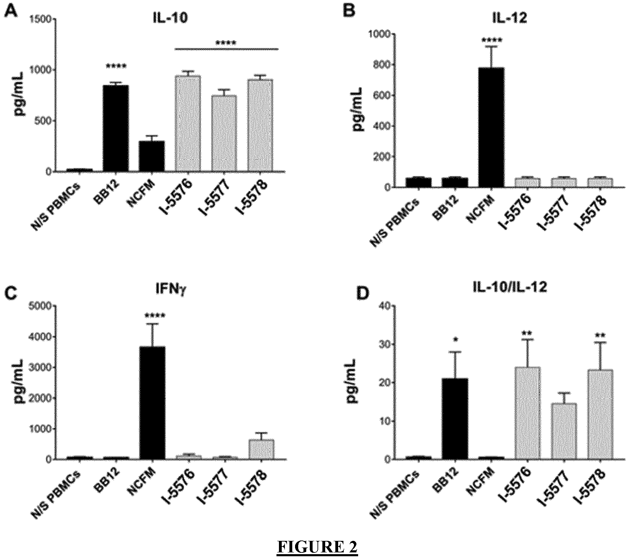

Figure 2 illustrates *in vitro* immunomodulatory profiles of the different strains. **(A)** IL-10, **(B)** IL-12, **(C)** IFN-γ production (in pg/mL) was evaluated in the supernatants of PBMCs (n= 5 different donors) stimulated for 24h by the tested strains (CNCM 1-5576 and CNCM 1-5578 according to the invention, or CNCM 1-5577 outside the invention) or two control strains (*L. acidophilus* NCFM (NCFM) known as a pro-Th1 strain and *B. animalis subsp. lactis* BB12 (BB12) known as an anti-inflammatory strain), in comparison to non-treated cells (N/S PBMCs). (D) IL-10/IL-12 ratio was calculated for each of the supernatants. Data represent means ± SEM of the 5 independent donors. * refers to the comparison of bacteria-stimulated PBMCs versus untreated cells; *p < 0.05, ***p < 0.001, ****p < 0.0001.

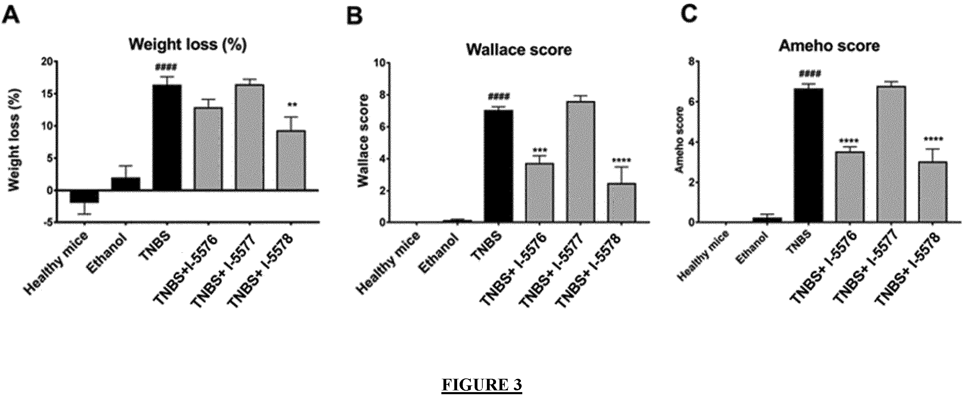

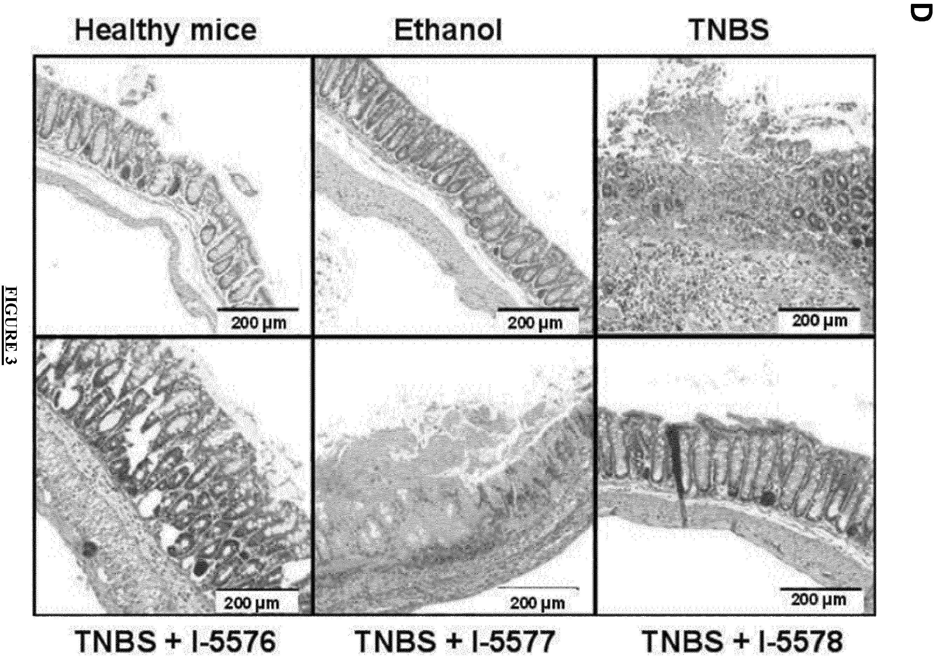

Figure 3 illustrates the ability of *P. distasonis* strains (CNCM 1-5577 strain outside the invention or CNCM 1-5576 and CNCM 1-5578 strains according to the invention) to alleviate the severity of acute TNBS-induced colitis. Colitis was induced by intrarectal administration of TNBS dissolved in 0.9% NaCl/ethanol solution (50/50 v/v) at a dose of 95 mg/kg. A group of mice was treated with the solvent alone (50% ethanol, "ethanol" mice) and a group of untreated mice was also included (control "healthy mice"). **(A)** Body weight loss (as a percentage of the initial weight). **(B)** Macroscopic evaluation of colonic inflammation (Wallace score). **(C)** Histologic evaluation of colonic inflammation (Ameho score). **(D)** Representative histological sections (stained by H&E, 100X magnification) of mice treated with TNBS (TNBS) or not (Healthy mice, Ethanol-control mice) and orally treated with the three selected strains. The scale indicated on each microscope observation is 200 µm. Data represent means of each group (n = 9 mice per group) ± SEM. # and * refer to the comparisons of TNBS versus healthy mice or bacteria-treated group versus TNBS control group, respectively; **p < 0.01, *** p < 0.001, #### or **** p < 0.0001.

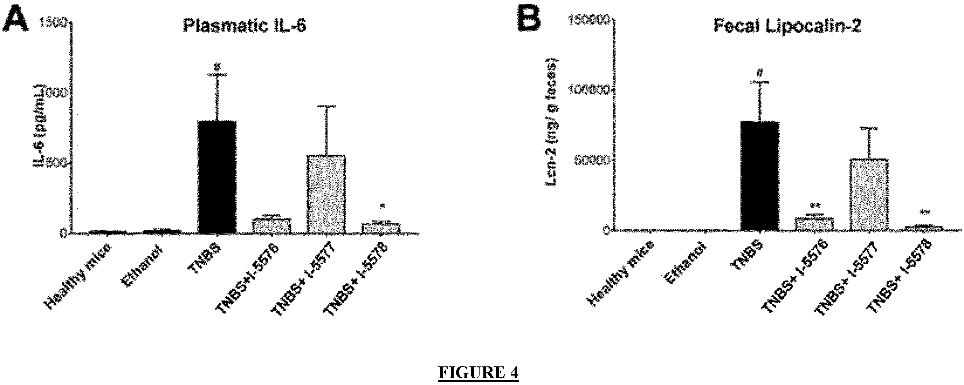

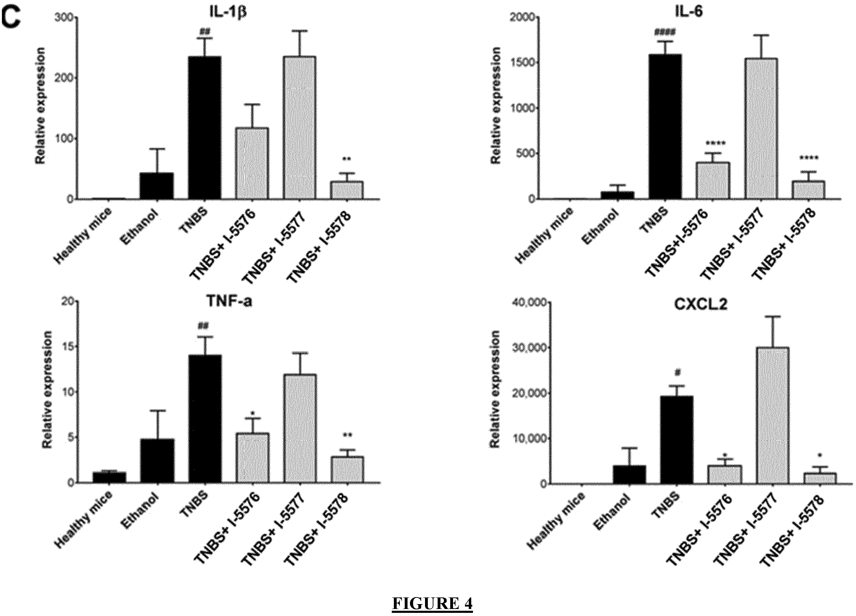

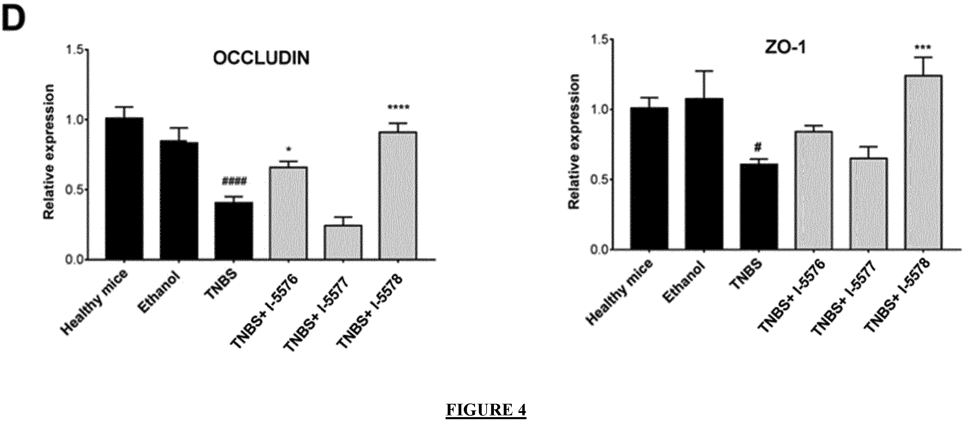

Figure 4 illustrates the capacity of the strains (CNCM 1-5577 strain outside the invention or CNCM 1-5576 and CNCM 1-5578 strains according to the invention) to modulate **(A)** the plasmatic IL-6 concentration (pg/mL), **(B)** the fecal lipocalin-2 levels (µg/g feces), **(C)** the expression of genes encoding pro-inflammatory markers (*IL-1β, IL-6, TNF-α* and *CXCL2)* or **(D)** tight junction proteins during TNBS-induced colitis. IL-6 and fecal lipocalin-2 concentrations were measured by ELISA. Gene expression of *IL-1β, IL-6, TNF-α, CXCL2,* Occludin and Zo1 was evaluated by qRT-PCR from colonic samples obtained two days after colitis induction. Colitis was induced by intrarectal administration of TNBS dissolved in 0.9% NaCl/ethanol solution (50/50 v/v) at a dose of 95 mg/kg. A group of mice was treated with the solvent alone (50% ethanol, control "ethanol mice") and a group of untreated mice was also included (control "healthy mice"). Results are expressed as Relative expression compared with values obtained from healthy mice. Data represent means of each group (n = 9 mice per group) ± SEM. # and * refer to the comparisons of the TNBS-treated control versus healthy mice or bacteria-treated group versus TNBS control group, respectively; #or* p < 0.05, ##or** p < 0.01, *** p < 0.001, #### or **** p < 0.0001.

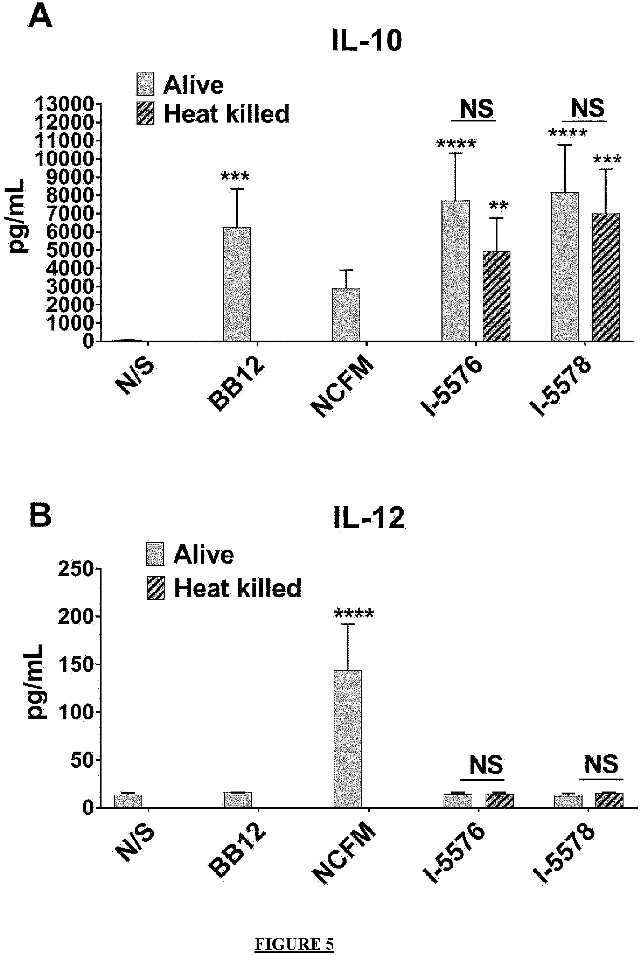

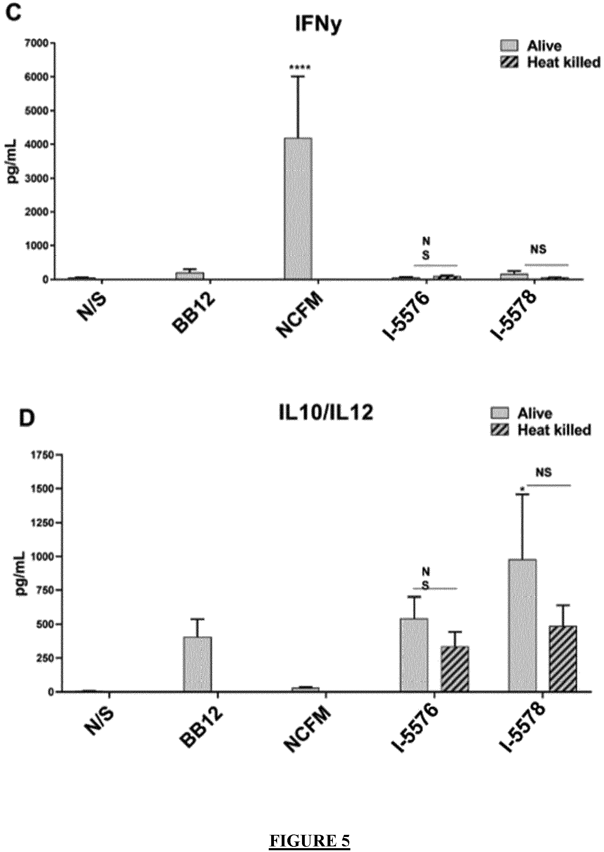

Figure 5 illustrates *in vitro* immunomodulatory profiles of the strains of the invention before or after they are inactivated by thermization. **(A)** IL-10, **(B)** IL-12, **(C)** IFN-γ production (in pg/mL) was evaluated in the supernatants of PBMCs (n= 5 different donors) stimulated for 24h by the tested strains (CNCM 1-5576 and CNCM 1-5578 according to the invention) or two control strains (*L. acidophilus* NCFM (NCFM) known as a pro-Th1 strain and *B. animalis subsp. lactis* BB12 (BB12) known as an anti-inflammatory strain), in comparison to non-treated cells (N/S PBMCs). **(D)** IL-10/IL-12 ratio was calculated for each of the supernatants. Data represent means ± SEM of the 4 independent donors. * refers to the comparison of bacteria-stimulated PBMCs (alive or heat killed) versus untreated cells; *p < 0.05, ****p < 0.001, and NS (non significant) refers to the comparison of bacteria alive versus heat killed bacteria.

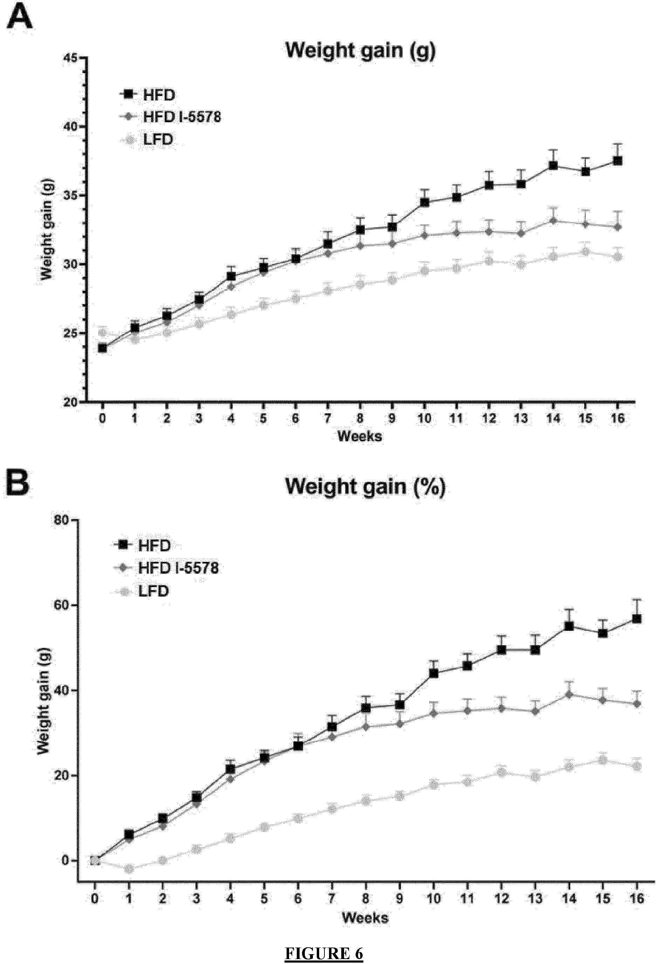

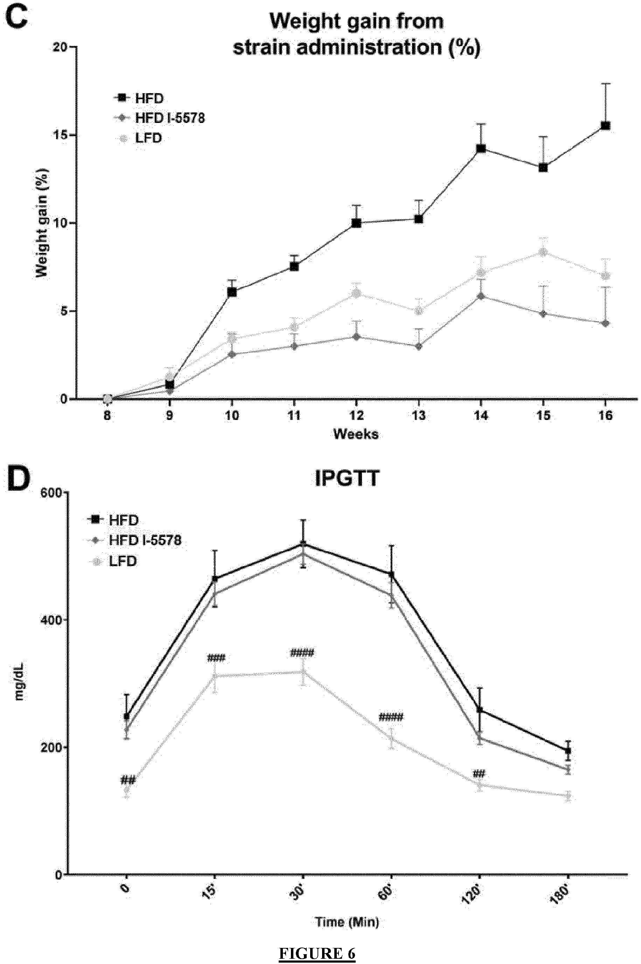

Figure 6 illustrates the ability of *P. distasonis* strain CNCM 1-5578 according to the invention to impact on the development of High fat diet (HFD)-induced obesity in mice. Mice (n = 13 mice per group) were randomly assigned to be fed either with low fat diet (LFD) or high fat diet (HFD). After 8 weeks, mice were mainained on diet and received a daily oral administration of 10⁹ colony-forming units (CFU) of the CNCM 1-5578 strain resuspensed in sterile PBS or PBS alone (control LFD and HFD mice), five consecutive days per week, for additional 8 weeks **(A)** Evolution of the body weights (in g) of mice receiving the CNCM I-5578 strain or PBS and fed with LFD (low fat diet) or HFD diet for 16 weeks. **(B)** Evolution of the body weight gains (expressed as a percentage of the body weight on day 0 of diet intervention) of mice receiving the CNCM 1-5578 strain or PBS and fed with LFD or HFD diet for 16 weeks. **(C)** Evolution of the body weight gains (expressed as a percentage of the body weight on day 0 at the day of starting treatment with the strain) of mice receiving the CNCM 1-5578 strain or PBS and fed with LFD or HFD diet for 16 weeks **(D)** Glucose tolerance test 12 weeks post-diet. Blood glucose levels (mg.dl⁻¹) were measured after intraperitoneal glucose injection. Area under the cuves (AUC) values were calculated and included at the top of each graph. Data represent means of each group (n = 13 mice per group) ± SEM. # and * correspond to regimen effect (HFD vs LFD) and treatment effect (CNCM 1-5578 vs PBS in HFD), respectively. *p < 0.05, *** or #### p < 0.001, **** p < 0.0001.

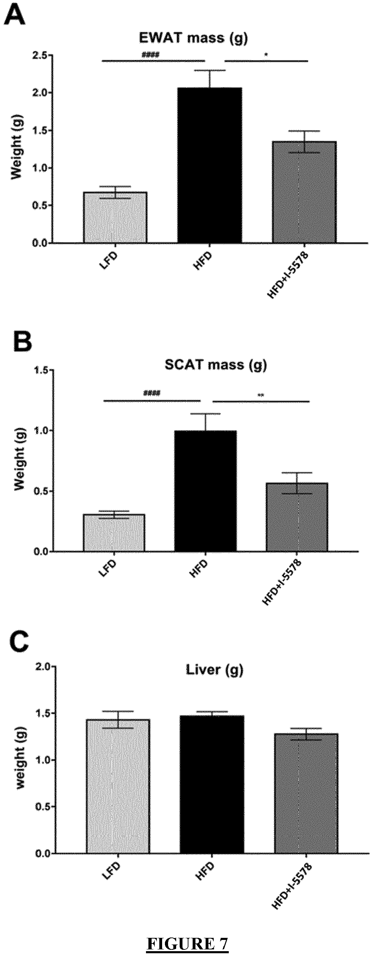

Figure 7 illustrates the impact of *P. distasonis* strain CNCM 1-5578 according to the invention on the development of fat depots and modification of the liver in High fat diet (HFD)-induced obesity in mice. Mice (n = 13 mice per group) were randomly assigned to be fed either with low fat diet (LFD)) or high fat diet (HFD). After 8 weeks, mice were mainained on diet and received a daily oral administration of 10⁹ colony-forming units (CFU) of the CNCM 1-5578 strain resuspensed in sterile PBS or PBS alone (control LFD and HFD mice), five consecutive days per week, for additional an 8 weeks. **(A)** Epididymal adipose tissue (EWAT) mass at the end of the experiment (in g). **(B)** Subcutaneous adipose tissue (SCAT) mass at the end of the experiment (in g). **(C)** Liver mass at the end of the experiment (in g). Data represent means of each group (n = 13 mice per group) ± SEM. # and * correspond to regimen effect (HFD vs LFD) and treatment effect (1-5578 vs PBS in HFD), respectively. *p < 0.05; ** p < 0.01; #### p < 0.001.

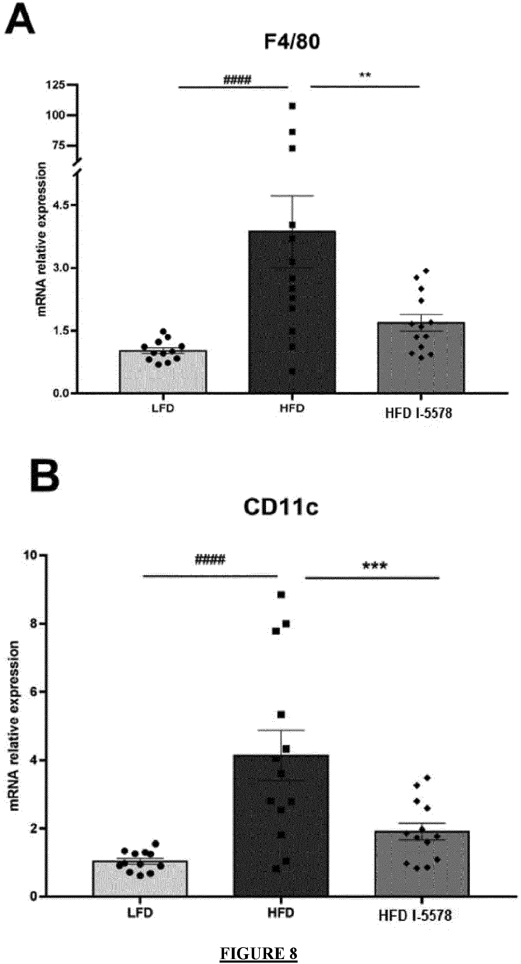

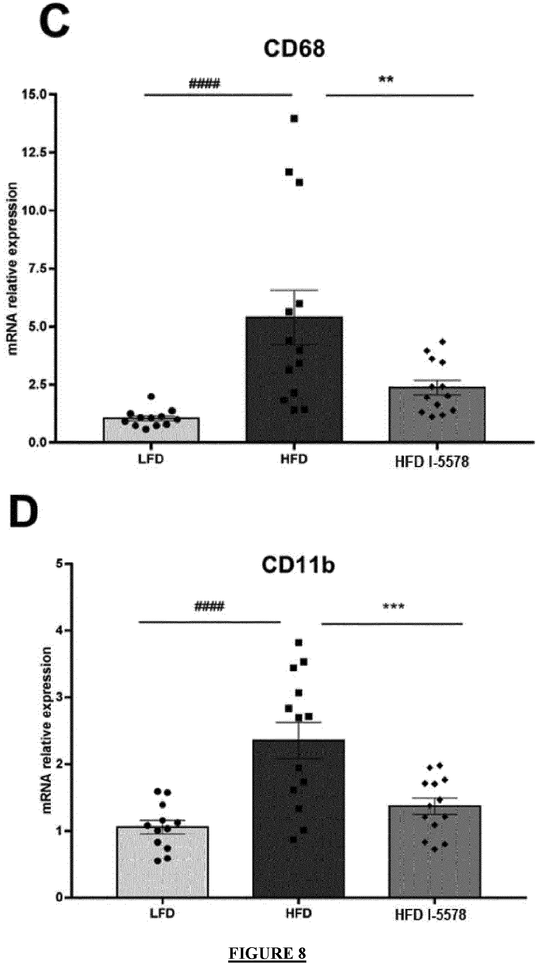

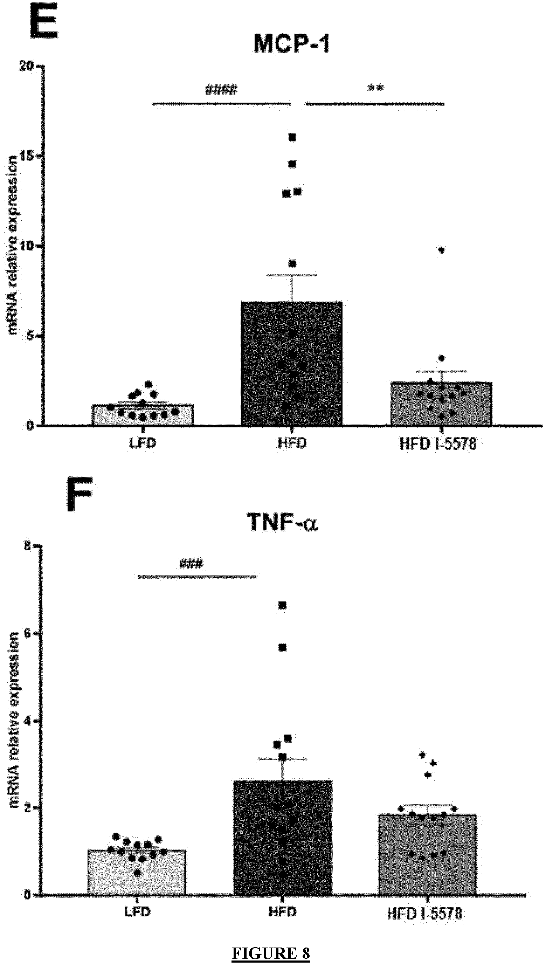

Figure 8 illustrates the impact *of P. distasonis* strain CNCM 1-5578 according to the invention on the local inflammation in the visceral adipose tissues, as measured by the expression of inflammatory genes (TNF-α (Figure 8F), MCP-1 (Figure 8E)) and genes corresponding to specific markers of monocyte/macrophage recruitment (F4/80 (Figure 8A), CDllb (Figure 8D), CD11c (Figure 8B), CD68 (Figure 8C)). Mice (n = 13 mice per group) were randomly assigned to be fed either with low fat diet (LFD)) or high fat diet (HFD). After 8 weeks, mice were mainained on diet and received a daily oral administration of 10⁹ colony-forming units (CFU) of the CNCM 1-5578 strain resuspensed in sterile PBS or PBS alone (control LFD and HFD mice), five consecutive days per week, for additional an 8 weeks. Data represent means of each group (n = 13 mice per group) ± SEM. # and * correspond to regimen effect (HFD vs LFD) and treatment effect (1-5578 vs PBS in HFD), respectively. *p < 0.05; ** p < 0.01; #### p < 0.001.
   Ordinate: mRNA relative expression
   Abscissa: from left to right: LFD, HFD and HFD I-5578.

### Detailed description of the invention

In the context of the present invention, the term " prevent" denotes the reduction to a lesser degree of the risk or of the probability of occurrence of a given phenomenon, that is to say, in the present invention, a gastrointestinal inflammation, in particular a bowel inflammation.

As used herein, the terms "*treating*" or "*treat*" include alleviation of the symptoms associated with a specific disorder or condition and/or elimination of said symptoms.

An "*infant*" as defined herein is a human child from birth to the end of the first year of life.

A "*child*" as defined herein is a human child between infancy and puberty. Puberty, which is the process of physical changes through which a child's body matures into an adult body capable of sexual reproduction, may be reached at different ages for different children. Generally, a child may be defined as a human child between the ages of one and 10 for a female child, and between one and fifteen for a male child.

A "*newborn infant*" or "*neonate*" is a human infant from the time of birth through the 28th day of life

A "*premature infant*" as used herein is one born before a gestational age of 37 completed weeks (259 days). The duration of gestation is measured from the first day of the last menstrual period and is expressed in completed days or weeks.

Finally, an "*infant born by caesarean*" is an infant that is delivered by the use of surgery, called a caesarean section, or C-section, or caesarean delivery. A caesarean section is an alternative to vaginal delivery, and is often necessary when vaginal delivery is too risky for the baby or the mother (e.g. obstructed labor, twin pregnancy, high blood pressure in the mother, breech birth, or problems with the placenta of umbilical cord).

By "*obesity*" is meant a pathophysiological condition in which an individual has, in particular, an excess of adipose tissue, generally induced by an obesogenic diet, comprising, in particular, excessive caloric consumption, genetic predispositions or an insufficient or non-existent practice of sports. An individual declared as obese has a body mass index (BMI) greater than 30. The body mass index, according to an official definition of the World Health Organization (WHO), is the indicator of health risks associated with overweight and underweight. BMI is calculated by dividing the weight of the individual (in kilograms) by their height (in meters) squared. A BMI value is associated with a specific body size according to the classification given by the WHO.

The term "*physiologically acceptable medium*" is intended to denote a medium which is compatible with the body of the individual to whom said composition must be administered. It is, for example, a non-toxic solvent such as water. In particular, said medium is compatible with oral administration.

The terms "*next generation probiotics*" or "*pharmabiotics*" as defined herein refer to a live commensal microorganism, or their products, that present a proven pharmacological role in health or in treating and/or preventing diseases.

The inventors investigated the health-promoting effects of particular *Parabacteroides distasonis* strains by combining:
- *in vitro* approaches to evaluate their abilities to strengthen the epithelial barrier and to reduce and/or prevent a gastrointestinal inflammation and;
- *in vivo* studies confirming the advantageous properties of the strains according to the invention:
- in an appropriate murine model of colitis; and
- in an appropriate model of mice whose obesity is induced by a High Fat Diet (HFD);
demonstrating the strong abilities of the two strains of the invention, supporting their use in the management of IBD and associated diseases, in particular obesity.

Indeed, the inventors have determined, unexpectedly, that the *P. distasonis* strains CNCM 1-5576 and CNCM 1-5578 have the capacity to reduce a gastrointestinal inflammation, in particular a bowel inflammation, or a disorder associated with gastrointestinal inflammation, in an individual.

### Parabacteroides distasonis strains of the invention

*Parabacteroides distasonis* (previously known as *Bacteroides distasonis*) is a Gram-negative, non-spore forming, anaerobic, rod-shaped, and non-motile bacterium from the genus of *Parabacteroides.*

*P. distasonis* is a member of the normal distal human gut microbiota. The distal gut microbiota contains more bacterial cells than all of our body's other microbial communities combined. More than 90% of phylogenetic types belong to two phyla, the Bacteroidetes and the Firmicutes, with the remaining types distributed among four other divisions. *P. distasonis* has the smallest genome (accession number NC_009615) among the sequenced human gut-associated Bacteroidetes, the smallest repertoire of genes involved in environmental sensing and gene regulation and the smallest number of genes associated with carbon source degradation. It lacks many accessory hemicellulases, pectinases and polysaccharidases that target non-plant carbohydrates. Of note, there are currently 12 known species belonging to the *Parabacteroides* genus.

The *P. distasonis* strains CNCM 1-5576 and CNCM 1-5578 of the invention have been isolated from neonates gut microbiota.

As previously indicated, the anti-inflammatory properties of the CNCM 1-5576 and CNCM 1-5578 strains of the invention cannot generally be attributed to the *P. distasonis* species, given that the existence of anti-inflammatory properties is unpredictable for a given strain of *P. distasonis.* This is clearly illustrated in the examples of the present text, in which a comparative test was carried out with a *P. distasonis* strain not part of the invention, namely the CNCM 1-5577 strain, also isolated from neonate gut microbiota, which does not show the same advantageous properties as the strains of the invention.

The present invention accordingly relates to the bacterial strain deposited with the CNCM under accession number CNCM 1-5576 and to the bacterial strain deposited with the CNCM under accession number CNCM 1-5578, *per se.*

The present invention also relates to a *Parabacteroides distasonis* strain of the invention, selected from the group consisting of the bacterial strain deposited with the CNCM under accession number CNCM 1-5576 and the bacterial strain deposited with the CNCM under accession number CNCM 1-5578, is for use thereof in the treatment and/or prevention of a gastrointestinal disease in an individual, in particular of a gastrointestinal inflammation, and more particularly of a bowel inflammation in an individual.

A *Parabacteroides distasonis* strain of the invention, selected from the group consisting of the bacterial strain deposited with the CNCM under accession number CNCM I-5576 and the bacterial strain deposited with the CNCM under accession number CNCM I-5578, is for use thereof in the treatment and/or prevention of a disease associated with a gastrointestinal disease, and in particular associated with a gastrointestinal inflammation.

More particularly, a *Parabacteroides distasonis* strain of the invention, selected from the group consisting of the bacterial strain deposited with the CNCM under accession number CNCM 1-5576 and the bacterial strain deposited with the CNCM under accession number CNCM 1-5578, is for use thereof in the treatment and/or prevention of:
- inflammatory gastrointestinal diseases;
- disorders associated with an inflammatory gastrointestinal disease; and/or
- a leaky gastrointestinal mucosal barrier.

The bacterial strain of the invention is in a live, semi-active, inactivated, such as inactivated by heat, and/or dead form, and in particular in a live form or in an inactivated by heat form.

Within the meaning of the invention, a bacterial strain in a semi-active form is a microorganism whose capacity to proliferate is reduced, temporarily or permanently.

The term "*semi-active*" thus designates a bacterium with low physiological activity. This activity can be measured by an exponential growth phase or a longer generation time, a slower metabolism or an incomplete physiological response to changes in the environment, for example. In some extreme cases, the number of bacteria can be reduced since they can no longer resist changes in the environment.

Thus, within the meaning of the invention, an "*inactivated*" bacterial strain is a bacterial strain which is no longer able, temporarily or permanently, to proliferate.

Within the meaning of the invention, a "*dead*" bacterial strain is a bacterial strain which is no longer capable, definitively, of proliferating.

Dead or inactivated bacterial strains can have intact or ruptured cell membranes. Accordingly, the term "*inactivated*" also designates the extracts and lysates of bacterial strains. Obtaining dead or inactivated bacterial strains can be carried out by any method known to those skilled in the art.

An inactivated bacterial strain suitable for the invention can be prepared by irradiation, thermal inactivation also called inactivation by heat or heat killed, or lyophilization of a preparation of bacterial strain. These methods are known to those skilled in the art.

In a particular embodiment, a strain of the invention is administered in a live form.

The bacterial strains of the invention may be administered to the intestines of an individual in need thereof in various ways, and in particular orally or rectally. Bacterial strains according to the invention are in particular administered orally.

According to a particular embodiment, the bacterial strains of the invention are in a composition comprising a physiologically acceptable medium.

### Compositions

The present invention also relates to a composition comprising, in a physiologically acceptable medium, at least a *Parabacteroides distasonis* strain of the invention, selected from the group consisting of the bacterial strain deposited with the CNCM under accession number CNCM 1-5576 and the bacterial strain deposited with the CNCM under accession number CNCM I-5578.

The present invention accordingly also relates to a bacterial strain of the species *Parabacteroides distasonis* of the invention for its use as mentioned above, the bacterial strain being in a composition comprising a physiologically acceptable medium.

A composition according to the invention is intended for the digestive tract, in particular the intestines.

Consequently, a composition according to the invention is in particular chosen from an oral or rectal composition, i.e. a composition according to the invention is in particular in an form appropriate for administration through the oral or rectal route to an individual. A composition of the invention is in particular an oral composition, i.e. it is intended for oral administration to an individual.

Such a composition may be in the form of a suspension, a tablet, a pill, a capsule, a granule or a powder.

The composition according to the invention for oral administration may in particular be chosen from the group consisting of a food product, a beverage, a pharmaceutical product, a nutraceutical, a food additive, a food supplement or a milk product and is, in particular, a food supplement.

According to one preferred embodiment, a composition according to the invention is a food supplement.

A food supplement for oral administration may be present in capsules, gel capsules, soft capsules, tablets, sugar-coated tablets, pills, pastes, lozenges, gums, oral solutions or emulsions, a syrup or a gel.

A food supplement according to the invention may also comprise a sweetener, a stabilizer, an antioxidant, an additive, a flavoring agent and/or a dye.

The formulation thereof is carried out by means of the usual methods for producing sugar-coated tablets, gel capsules, gels, controlled-release hydrogels, emulsions, tablets or capsules.

A composition according to the invention may also be in the form of a nutritional composition.

A nutritional composition according to the invention is in the form of a yogurt, a cereal bar, breakfast cereals, a dessert, a frozen food, a soup, a pet food, a liquid suspension, a powder, a tablet, a gum or a candy.

Advantageously, a composition according to the invention, intended for oral administration, may be provided with a gastric-juice-resistant coating, in order to ensure that the bacterial strain of the invention included in said composition can pass through the damaged stomach. The release of the bacterial strain may thus take place for the first time in the upper intestinal tract.

In another embodiment of the invention, a composition containing the bacterial strains of the invention is a rectal composition, i.e. it is intended to be intrarectally administered.

Such rectal administration can be carried out in the form of a suppository, an enema or a foam.

A composition of the invention can comprise one of the strains of the invention or the two strains of the invention.

In a particular embodiment, the composition of the invention comprises:
- the bacterial strain deposited with the CNCM under accession number CNCM I-5576; and
- the bacterial strain deposited with the CNCM under accession number CNCM I-5578.

In a particular embodiment, a composition of the invention is suitable for the administration of a daily dose representing from 10⁷ to 10¹¹ colony-forming units (cfu) as a medicament, preferably in the form of a daily dose equivalent to 10⁹ cfu of the bacterial strain(s) of the invention.

For example, a composition according to the invention may be administered to an individual requiring it, at a single daily dose of 1 g containing the bacterial strain deposited with the CNCM under accession number CNCM 1-5576, the bacterial strain deposited with the CNCM under accession number CNCM 1-5578 or a mixture thereof, in an amount equivalent to a dose of between 10⁷ and 10¹¹ cfu, preferably 10¹⁰ cfu.

In another example, a composition according to the invention may be administered to an individual requiring it, at a single daily dose of 0.2 g containing the bacterial strain deposited with the CNCM under accession number CNCM 1-5576, the bacterial strain deposited with the CNCM under accession number CNCM 1-5578 or a mixture thereof, in an amount equivalent to an amount of between 10⁷ and 10¹¹ cfu, preferably 10¹⁰ cfu.

In another example, a composition according to the invention may be administered to an individual requiring it, twice a day on the basis of two doses of 1 g, each dose containing, independently, the bacterial strain deposited with the CNCM under accession number CNCM 1-5576, the bacterial strain deposited with the CNCM under accession number CNCM 1-5578 or a mixture thereof in an amount equivalent to an amount of between 5×10⁶ and 5×10¹⁰ cfu (on the dry weight basis), preferably 5×10⁹ cfu, so that the total daily dose of the bacterial strain deposited with the CNCM under accession number CNCM 1-5576, the bacterial strain deposited with the CNCM under accession number CNCM 1-5578, or a mixture thereof administered to the individual is as indicated above.

A composition according to the invention may also comprise at least one from the list of ingredients consisting of: antioxidants, fish oils, DHA, EPA, vitamins, minerals, phytonutrients, a protein, a lipid, probiotics, prebiotics, postbiotics, polyphenols, flavonoids, and combinations thereof.

### Implementation of the strains and compositions of the invention

As previously mentioned, a bacterial strain of invention, as well as a composition comprising it, is for use thereof in the treatment and/or prevention of a gastrointestinal disease, or of a disorder associated with a gastrointestinal disease, in an individual.

More particularly, a a bacterial strain of invention, as well as a composition comprising it, is for use thereof in the treatment and/or prevention of:
- inflammatory gastrointestinal diseases;
- disorders associated with an inflammatory gastrointestinal disease; and/or
- a leaky gastrointestinal mucosal barrier.

An inflammatory gastrointestinal disease of the invention can in particular be an inflammatory bowel disease (IBD). In a particular embodiment, an IBD may be a colonic inflammatory bowel disease. In another embodiment, an IBD may be localised in other parts of the gastrointestinal tract, such as in ileal, ileocolonic or colonic sites.

Said inflammatory bowel disease may in particular be chosen from the group consisting of Crohn's disease, ulcerative colitis and pouchitis. It is more particularly chosen from the group consisting of Crohn's disease and ulcerative colitis.

In a particular embodiment, disorders associated with gastrointestinal inflammation/an inflammatory gastroinstestinal disease include:
- obesity, as body mass index and overweight are associated with low grade inflammation and intestinal barrier weakening and are emerging features of inflammatory gastrointestinal diseases, such as Crohn's disease;
- diabetes, as the incidence of diabetes in patients with inflammatory gastrointestinal diseases, in particular IBD, has been demonstrated to be significantly higher compared with subjects in the general population matched by age, sex, BMI, smoking, alcohol drinking, exercise and income;
- autism spectrum disorders (anxiety, autism, schizophrenia, neurodevelopment,...), as studies have demonstrated that children with autism spectrum disorders who experience gastrointestinal disorders are more likely to have more severe symptoms of autism spectrum disorders, and that treating or alleviating the gastrointestinal symptoms can relieve the behavioural and social symptoms of autism spectrum disorders;
- perinatal inflammation;
- bronchopulmonary dysplasia;
- retinopathy of prematurity;
- cystic fibrosis, which is associated with gut microbiota dysbiosis ; and
- colon cancer.

In a particular embodiment, disorders associated with gastrointestinal inflammation are also associated with gut microbiota dysbiosis. As such, these disorders are either caused by or responsible of causing gut microbiota dysbiosis.

Said disorder associated with an inflammatory gastrointestinal diserase is in particular obesity or diabetes, and is in particular obesity.

As previously mentioned, a bacterial strain of the invention, or a composition according to the invention comprising at least one bacterial strain of the invention, can also be used for the treatment and/or prevention of a leaky gastrointestinal mucosal barrier, also termed a leaky gut. A strain according to the invention, and in particular strain 1-5578, is indeed able, even in the absence of a local inflammation, to restore the integrity or limit the permeability of a gut barrier, and in particular to restaure the integrity of a gut barrier characterized by a permeability which is superior to the one of an healthy gut barrier.

As previously discussed, such property plays an important role in particular in both the prevention and the treatment of a gastrointestinal inflammation.

An individual to which a strain or composition of the invention is administered can in particular be an infant or a child, in particular a newborn infant, and in particular is a premature infant or an infant born by caesarean section.

Premature infants and/or infants born by caesarean are usually born with a more immature immune system than babies born by vaginal birth and/or at term.

What's more, premature infants have an immature gastrointestinal tract, with a gut epithelium that has diminished barrier function and increased permeability, rendering them more sensitive to developing an inflammatory gastrointestinal disease. Prematurity also tends to lead to lack of diversity in the gut bacterial communities including taxa frequently found to colonize the gut of healthy term infants, as well as bacteria that are associated with immune training, metabolic function, and ultimately decreased risk of lifelong health issues such as obesity (Jacquot, A. et al. Dynamics and clinical evolution of bacterial gut microflora in extremely premature patients. J. Pediatr. 158, 390-396 (2011), Arboleya, S. et al. Establishment and development of intestinal microbiota in preterm neonates. FEMS Microbiol. Ecol. 79, 763-772 (2012), Butel, M.-J. et al. Conditions of bifidobacterial colonization in preterm infants: a prospective analysis. J. Pediatr. Gastroenterol. Nutr. 44, 577-582 (2007) and Westerbeek, E. A. et al. The intestinal bacterial colonisation in preterm infants: a review of the literature. Clin. Nutr. 25, 361-368 (2006)).

In the case of infants born by caesarean, it is known that they carry different microbes from children born through the vaginal canal. Indeed, babies delivered by c-section fail to acquire some of the microbes from their mothers that vaginally delivered children gain. Newborns delivered by c-section tend to lack strains of commensal bacteria - those typically found in healthy individuals - whereas these bacteria make up most of the gut community of vaginally delivered infants. Instead, the guts of c-section babies are dominated by opportunistic bacteria such as *Enterococcus* and *Klebsiella,* which circulate in hospitals (Shao, Y. et al. Nature https://doi.org/10.1038/s41586-019-1560-1 (2019)). Months after birth, the infants' microbiotas usually grow more similar - with the exception of *Bacteroides.* These bacteria are absent or present at very low levels in the microbiotas of nearly all c-section babies after birth. Nine months later, on average, around 60% of these babies still harbour few or no *Bacteroides* in their guts (Dominguez-Bello, M. G. et al. Nature Med. 22, 250-253 (2016)).

As such, these individuals are particularly prone to developing inflammatory gastrointestinal diseases, which can more particularly lead to severe disorders, such as those mentioned above.

A strain according to the invention can be administered in combination with other medical treatments known for their ability to prevent and/or treat the targeted disorder or disease.

Such medical treatments are well known to one skilled in the art and may be selected by a medical practioner, in particular taking into account the age, the sex and the overall condition of the patient to be treated. Said medical treatments may include at least one ingredient selected from the group consisting of antioxidants, vitamins, minerals, phytonutrients, a protein, a lipid, probiotics, prebiotics, fibers, postbiotics, polyphenols, flavonoids, and combinations thereof. Mention may also be made of synthetic antiinflammatories, anti-alpha-TNF, inhibitors of signalling pathways, protease inhibitors, statins, etc.

Such medical treatments can be administered simultaneously or sequentially compared to a strain or a composition of the invention.

When said medical treatment consists at least partially in the administration of a medical active to the considered individual, said medical active and a strain according to the invention can be administered in the same composition or in separate compositions. When they are in separate compositions, the composition comprising a strain according to the invention and the composition comprising the medical active can be administered through the same route or through different routes.

By simultaneously, it is understood that the compositions can be administered at the same moment or up to the same day or couple of days.

By separately, it is understood that the compositions can be administered with at least several days, for example at least two days of difference.

The invention is described below in greater detail by means of the following examples which are given solely by way of illustration.

All the references to percentages are percentages by weight unless otherwise indicated.

### EXAMPLES

The *Parabacteroides distasonis* strains according to the invention, deposited with the CNCM under the accession numbers CNCM 1-5576 and CNCM 1-5578, were tested and compared to another *P. distasonis* strain, CNCM 1-5577, for their capacity to modulate, *in vitro* and *in vivo,* the immune response and have a direct impact on the gastrointestinal inflammation.

### I. Materials and Methods

### 1. Bacterial strains and growth conditions

Bacteria evaluated in the present study are listed in Table 1. *P. distasonis* strains were cultured at 37°C in Brain-heart infusion medium supplemented (BHIS) with yeast extract (0.5%, Difco), hemin (0.5 mg/mL; Sigma-Aldrich), maltose (0.5 mg/mL; Sigma-Aldrich), cellobiose (0.5 mg/mL; Sigma-Aldrich), cysteine (0.5 mg/mL; Sigma-Aldrich) and K1 vitamin (0.098 mg/L; Sigma-Aldrich) in an anaerobic chamber (Jacomex, Fr) supplied with BIO300 (Air Liquide, Fr). After centrifugation (6 000 rpm, 15 min at 4°C), Culture pellets were washed with phosphate buffered saline (PBS) (pH 7.2) maintained in anaerobiosis and bacteria were concentrated by centrifugation at 6,000 rpm for 15 min at 4°C.

For *in vitro* experiments, cell pellets were suspended at 10⁹ CFU/mL in anaerobic PBS containing 25% glycerol and suspensions were frozen in liquid nitrogen before storage at -80°C. For *in vivo* experiments, dry pellets (without PBS) were frozen in liquid nitrogen and stored at -80°C.

For heat-inactivated bacteria, bacterial suspensions were heated 30 min at 70°C. The absence of viability was checked by plating the suspension on medium. Suspensions were frozen as described for live bacteria until use for PBMC stimulation.

**Table 1. Bacterial strains, growth media and origins.**

| **Strain designations** | **Species** | **Growth medium** | **Origin** |
|---|---|---|---|
| CNCM 1-5576 | *Parabacteroides distasonis* | BHIS | Newborn fecal samples |
| CNCM 1-5577 | *Parabacteroides distasonis* | BHIS | Newborn fecal samples |
| CNCM 1-5578 | *Parabacteroides distasonis* | BHIS | Newborn fecal samples |

Two strains were used as **control strains** for the PBMC stimulation test. *Lactobacillus acidophilus* NCFM kindly provided by DuPont^{™} Danisco (Madison, WI, USA) was included as a pro-Th1 reference strain (Foligne, B. et al. World J. Gastroenterol. 2007, 13, 236-243) and was grown under limited aeration at 37°C in in De Man, Rogosa and Sharpe broth (MRS, Difco, Detroit, MI, USA). *Bifidobacterium animalis spp lactis* BB12 used as anti-inflammatory reference strain (Burns, P. et al. Sci Rep 2017, 7, 43211), was provided by Gabriel Vinderola (INLAIN, UNL-CONICET, Argentina) and grown anaerobically (GENbag anaer, Biomérieux, Marcy l'Etoile, France), in MRS supplemented with 0.05% L-cysteine-hydrochloride (Sigma, St-Louis, MO, USA). Bacteria were grown overnight, washed twice in sterile PBS buffer (pH 7.2) and resuspended to a final concentration of 10⁹ CFU/mL in PBS for *in vitro* studies.

### 2. In vitro epithelial barrier model

The human colon epithelial cell line Caco-2 clone TC7 (Chantret, I. et al. J. Cell. Sci. 1994, 107 (Pt 1), 213-225) was grown at 37°C with 10% CO2 in Dulbecco's modified Eagle Medium (DMEM, Life technologies, Grand Island, USA) supplemented with 5% heat-inactivated foetal calf serum, 1% non-essential amino acids, 2 mM glutamine (Gibco). 100 U/mL penicillin and 100 µg/mL streptomycin, as previously described (Alard, J. et al. Benef Microbes 2018, 9, 317-331). For the permeability assay, cells were grown on 12-well Transwell^{®} insert filter (polycarbonate membrane with 0.4 µm pore size, 12 mm diameters; Costar, Corning Life Science, Kennebunk, USA) at a density of 105 cells per cm2. The medium was changed every two days until day 17 when optimal trans-epithelial resistance was reached (TEER ≥ 1800 Ω/cm2), measured using a millicell-ERS (Electrical Resistance System; Millipore, Billerica, USA).

Cells were then incubated for 30 min in fresh DMEM medium and subsequently treated (or not) in the apical compartment, with bacteria at a bacteria-to-cell ratio of 10:1 (according to the number of cells expanded at that time point). Thirty minutes after, cells were sensitized with hydrogen peroxide (H2O2), in both apical and basal compartment (at 100 µM final concentration). Three different experiments were performed including duplicates of each condition. The results were compared to non-treated cells. TEER was measured just before H₂O₂ addition (T0) and every 30 min until 180 min. Results were expressed in % TEER compared to T0.

### 3. In vitro immunomodulation assay

Experimental protocols were approved by the Institut Pasteur de Lille committees in accordance with relevant guidelines and regulations. Blood samples were obtained from five healthy informed donors upon approved agreement (signed consents) by authorized staff. Peripheral blood mononuclear cells (PBMCs) were isolated from the blood as described before (Foligne et al., World J. Gastroenterol. 2007, 13, 236-243). Briefly, after Ficoll gradient centrifugation (GE Healthcare Bio-Sciences, Uppsala, Sweden), PBMCs were recovered at the interface, washed in RPMI-1640 medium (Gibco, Life Technologies, Ghent, Belgium), and adjusted to 2×10⁶ cells per ml in RPMI supplemented with gentamicin (150 µg/ml), glutamine (2 mM), and 10% heat-inactivated foetal calf serum (Gibco, Life Technologies, Grand Island, USA). PBMCs were stimulated with PBS or bacteria at a bacteria-to-cell ratio of 10:1. After 24 h of stimulation at 37°C under 5% CO2, supernatants were collected, clarified by centrifugation and stored at -20°C until cytokine measurements performed using R&D Duoset ELISA kits (R&D, Minneapolis, USA).

### 4. Murine model of 2,4,6-trinitrobenzenesulfonic acid (TNBS)-induced colitis

Housing and experimentations were performed in compliance with European guidelines of laboratory animal care (number 86/609/CEE) and French legislation (Government Act 87-848), approved by national and local Animal Ethics Committees (Nord-Pas-de-Calais CEEA N°75, Lille, France) and the Ministère de l'Education Nationale, de l'Enseignement Supérieur et de la Recherche, France.

Female BALB/C ByJ mice (7-8 weeks old) were purchased from Charles River (L'Arbresle, France). Mice were housed in specific pathogen-free condition at the animal facility of the Institut Pasteur de Lille and maintained in a temperature-controlled (20 ± 2°C) environment and a 12-h light/dark cycle. Mice were grouped in 6 animals/cage (n = 9 mice per group) and were given *ad libitum* access to regular mouse chow (Safe, Augy, France) and water. After one-week acclimation, the animals received daily an intragastric administration of bacteria (1 x 10⁹ CFU/ mice) in 200 µL gavage buffer or gavage buffer as control (200 mM NaHCO3 containing 1% glucose), starting 5 days before until 1 day after colitis induction. Control mice (healthy mice and TNBS control mice) received only gavage buffer.

A standardized TNBS-induced murine model of acute colitis was performed as previously described (Foligne, B. et al. World J. Gastroenterol. 2007, 13, 236-243 and Foligné, B. et al. Dig. Dis. Sci. 2006, 51, 390-400). Briefly, anesthetized mice received an intra-rectal administration (50 µL) of TNBS (Sigma-Aldrich Chemical, France) dissolved in 0.9% NaCl/ethanol (50/50 v/v) at a final concentration of 95 mg/kg. A group of mice was treated with the solvent alone (50% ethanol, control "ethanol" mice) and a group of untreated mice (control "healthy mice") was also included. Mice were weighed prior to TNBS administration and at sacrifice performed 48h after colitis induction. Blood samples were obtained by retro-orbital bleeding just before. Serum was stored at -20°C until measurement of IL-6 by ELISA (Duoset ELISA kits, R&D, Minneapolis, USA). Colons were removed, washed and carefully opened and macroscopic inflammation grading was performed blindly according to the Wallace criteria (Wallace, J.L. et al. Gastroenterology 1989, 96, 29-36), reflecting both the intensity and the extent of the inflammatory lesions. Histological analysis was performed on Hematoxylin & Eosine (H&E) stained 5-µm tissue sections from colon samples fixed in 4% formaldehyde and embedded in paraffin and blindly scored according to the Ameho scoring method (Ameho, C.K. et al. Gut 1997, 41, 487-493). Colonic samples were immediately stored in RNAlater^{®} buffer (Ambion, Life Technologies, Foster City, CA, USA) at -80°C until qRT-PCR analysis.

### 5. Quantification of fecal lipocalin 2 (Lcn-2)

Fresh fecal samples were collected 48 h after colitis induction and homogenized using Lysing Matrix D (MPbio, Eschwege, Germany) in PBS containing 0.1% Tween 20 (100 mg/mL). The samples were centrifuged for 10 min at 12,000 rpm at 4 °C and clear supernatants were collected and stored at -20°C until analysis, as reported by Chassaing et al (PLoS ONE 2012, 7, e44328). Supernatants were diluted from 50-fold to 10 000-fold depending on severity of the colitis and Lcn-2 levels were measured by ELISA using the Duoset kit (R&D System, Minneapolis, MN, USA), according to the manufacturer's instructions.

### 6. Real-time quantitative PCR (qRT-PCR)

Colonic tissue samples were homogenized using Lysing Matrix D (MPbio, Eschwege, Germany). Total RNA was extracted using Macherey-Nagel NucleoSpin RNAII isolation kit (Düren, Germany) according to the manufacturer's recommendation. Quantity and quality of RNA was checked by Nanodrop (260/280 nm, 260/230 nm). Complementary DNA was prepared by reverse transcription of 1 µg total RNA using High Capacity cDNA Reverse Transcription Kit (Applied Biosystems, Woolston Warrington, UK). qRT-PCR was performed using the Power SYBR Green PCR Master Mix (Applied Biosystems) on the QuantStudio^{™} 12K Flex Real-Time PCR System (Applied Biosystems, New Jersey, USA). All the results were normalized with the house keeping gene TATA-box-binding protein (Tbp).

Primers sequences are presented in table 2 below. Results are expressed as 2-ΔΔct values as described previously (Alard, J. et al. Benef Microbes 2018, 9, 317-331).

**Table 2. Forward and reverse primers used in the study for the respective genes encoding TATA-Box Binding Protein, IL-1β, IL-6, TNF-α, CXCL-2, ZO-1, Occludin, CD11c, CD11b, F4/80, MCP1, TNF and CD68.**

| | | |
|---|---|---|
| SEQ ID NO. 1 | Tbp-F | 5'-TGGTGTGCACAGGAGCCAAG-3' |
| SEQ ID NO. 2 | Tbp-R | 5'-TTCACATCACAGCTCCCCAC-3' |
| SEQ ID NO. 3 | Il1b-F | 5'-TTGACGGACCCCAAAAGATG-3' |
| SEQ ID NO. 4 | Il1b-R | 5'-AGAAGGTGCTCATGTCCTCA-3' |
| SEQ ID NO. 5 | i16-F | 5'-AGCCAGAGTCCTTCAGAGAGATAC-3' |
| SEQ ID NO. 6 | il6-R | 5'-ATTGGATGGTCTTGGTCCTTAGC-3' |
| SEQ ID NO. 7 | tnfa-F | 5'-CCCTCACACTCAGATCATCTTCTC-3' |
| SEQ ID NO. 8 | tnfa-R | 5'-GGCTACAGGCTTGTCACTCG-3' |
| SEQ ID NO. 9 | cxcl2-f | 5'-CAAAAGATACTGAACAAAGGCAA-3' |
| SEQ ID NO. 10 | cxcl2-R | 5'-TCAGGTACGATCCAGGCTTCC-3' |
| SEQ ID NO. 11 | zo1-F | 5'-GACTCCAGACAACATCCCGAA-3' |
| SEQ ID NO. 12 | zo1-R | 5'-ACGCTGGAAATAACCTCGTTC 3' |
| SEQ ID NO. 13 | occludin-F | 5'-TCAGGGAATATCCACCTATCACTTCAG-3' |
| SEQ ID NO. 14 | occludin-R | 5'-CATCAGCAGCAGCCATGTACTCTTCAC-3' |
| SEQ ID NO. 15 | CD11c-F | 5'-CCTGAGGGTGGGCTGGAT-3' |
| SEQ ID NO. 16 | CD11c-R | 5'-GCCAATTTCCTCCGGACAT-3' |
| SEQ ID NO. 17 | CD11b-F | 5' -AAACCACAGTCCCGCAGAGA-3' |
| SEQ ID NO. 18 | CD11b-R | 5'-CGTGTTCACCAGCTGGCTTA-3' |
| SEQ ID NO. 19 | F4/80-F | 5'-CACATCCAGCCAAAGCAGAA-3' |
| SEQ ID NO. 20 | F4/80-R | 5'-GTGTCTCGGATGCTTCCACAAT-3' |
| SEQ ID NO. 21 | MCP1-F | 5'-TCAGCCAGATGCAGTTAACGC-3' |
| SEQ ID NO. 22 | MCP1-R | 5'-TGATCCTCTTGTAGCTCTCCAGC-3' |
| SEQ ID NO. 23 | CD68-F | 5'-TGGCGGTGGAATACAATGTG-3' |
| SEQ ID NO. 24 | CD68-R | 5'-GATGAATTCTGCGCCATGAA-3' |

### 7. Murine model of HFD-induced obesity

C57BL/6J mice (males of 5-week-old) were purchased from Janvier Laboratories (L'Arbresle, France). Animals were housed in specific pathogen-free conditions in the animal facilities of the Institut Pasteur de Lille (accredited no. A59107) and maintained in a temperature-controlled (20 ± 2°C) facility with a strict 12-hour dark/light cycle. Housing and experimentations were performed in compliance with current national and institutional regulations and ethical guidelines (accredited no. APAFIS#22499-2019101811141602v2 from the Ministère de l'Education Nationale, de l'Enseignement Supérieur et de la Recherche, France). Before experimentation, animals were provided a one week acclimatisation period and were given *ad libitum* access to regular mouse chow and water. High-fat and control diets were purchased from Research Diets (Brogaarden, Lynge, Denmark) (respectively, HFD: 45% kcal fat; D12451, and LFD: 10% kcal fat; D12450H, both irradiated). Mice were randomly assigned to be fed either with LFD diet or HFD diet (n = 13 mice per group). After 8 weeks, mice were mainained on diet and received a daily oral administration of 10⁹ colony-forming units (CFU) of the CNCM 1-5578 strain resuspensed in sterile PBS or PBS alone (control LFD and HFD mice), five consecutive days per week, for additional 8 weeks. Body weights were weekly recorded. Intraperitoneal glucose tolerance (IP-GTT) test was performed as previously described (Alard *et al.,* 2016) after 12 weeks of diet. Sub-cutaneous (SCAT), epididymal (EWAT) adipose tissues, liver were collected and weighted at sacrifice. Blood, intestinal segments, caecal contents and fresh feces were also collected.

### 8. Real-time quantitative PCR (qRT-PCR)

Epididymal (EWAT) adipose tissue samples were homogenized using Lysing Matrix D (MPbio, Eschwege, Germany). Total RNA was extracted using Macherey-Nagel NucleoSpin RNAII isolation kit (Düren, Germany) according to the manufacturer's recommendation. Quantity and quality of RNA was checked by Nanodrop (260/280 nm, 260/230 nm). RNA (1 µg) was reverse-transcribed using the High capacity cDNA reverse transcription kit (Applied BiosystemsTM, Foster City, USA). Real-time quantitative PCR (RT-qPCR) was performed using the Power SYBR Green PCR Master Mix on the QuantStudio^{™} 12K Flex Real-Time PCR System (Applied Biosystems, New Jersey, USA). Primers used in the study are indicated in Table S2. The 2^{-ΔΔCT} method was used to normalize gene expression. All the results were normalized with the house keeping gene TATA-box-binding protein (Tbp).

Primers sequences are presented in table 2 above. Results are expressed as 2-ΔΔct values as described previously (Alard, J. et al. Benef Microbes 2018, 9, 317-331).

### 9. Statistical analysis

GraphPad Prism software was used for graph preparation and statistical evaluation. Differences between groups were assessed using ANOVA, followed by nonparametric Mann-Whitney test. Data with p value ≤ 0.05 were considered to be significant. The Pearson correlation coefficient was used to analyse correlation between lipocalin-2 and inflammation scores

### II. Results

### 1. P. distasonis strains displayed different ability to restore the H₂O₂-induced disruption of the epithelial barrier

The inventors evaluated the ability of three selected strains of the *P. distasonis* species to restore or strengthen the gut barrier function, by using an *in vitro* epithelial barrier model as previously described (Alard, J. et al. Benef Microbes 2018, 9, 317-331 and Seth, A. et al. Am. J. Physiol. Gastrointest. Liver Physiol. 2008, 294, G1060-1069). Strains CNCM I-5576 and CNCM 1-5578, according to the invention, and strain CNCM 1-5577, outside the invention, which served as a comparative strain, were used.

Polarized Caco-2 cell monolayers were exposed (or not) to the bacteria and were sensitized (or not) with H2O2.

Treatment of epithelial monolayers with H₂O₂ induced an increase in permeability, as measured by a decrease of the TEER in a time-dependent manner (Figure 1A). *P. distasonis* strain CNCM 1-5577 was not able to significantly restore the epithelial barrier (Figures 1A, B). On the contrary, the strains *P. distasonis* CNCM 1-5576 and CNCM 1-5578 were able to restore and even able to reinforce in a significant manner, the epithelial barrier, the TEER being higher than non-treated monolayers (Figure 1A, 1B).

### 2. P. distasonis strains CNCM 1-5576 and CNCMI-5578 exhibit the best anti-inflammatory in vitro profile

To investigate the immunomodulation capacities of the strains, human immune cells (PBMCs) were stimulated *in vitro* by the different bacteria (ratio 10:1) and their ability to induce the release of the anti-inflammatory IL-10 (Figure 2A) or the pro-Th1 IL-12 (Figure 2B) and IFNγ (Figure 2C) cytokines was measured by ELISA.

None of the strains induced a significant production of IL-12 (between 58 and 110 pg/mL) in comparison with untreated cells, while the pro-Th1 *L. acidophilus* NCFM reference strain induced significant levels (775 pg/mL).

All the *P. distasonis* were also low inducers of IFNγ (between 66 and 630 pg/mL), with levels much lower than those obtained for the pro-Th1 *L. acidophilus* NCFM strain (3655 pg/mL). All three strains induced a very high and significant (p < 0.001) level of IL-10 (between 744 and 936 pg/mL) with similar or higher inducing ability than the anti-inflammatory control strain *Bifidobacterium animalis spp lactis* BB12 (which induced 842 pg/mL IL-10).

However, as highlighted by the calculation of the IL-10/IL-12 ratio (Figure 2D), strains CNCM 1-5576 and CNCM 1-5578 exhibited an elevated and significant anti-inflammatory *in vitro* profile, while CNCM 1-5577 displayed the lowest anti-inflammatory effect.

On the basis of these *in vitro* results, the inventors observed that *P. distasonis* strains CNCM 1-5576 and CNCM 1-5578 exhibited the strongest anti-inflammatory profile together with a good ability to restore the epithelial barrier.

### 3. P. distasonis strains CNCM 1-5576 and CNCM 1-5578 were the most potent strains to counteract inflammation in a murine model of TNBS-induced colitis

The inventors evaluated the *in vivo* protective efficacy of the *P. distasonis* strains using a well-established murine model of acute colitis induced by a single rectal administration of TNBS (95 mg/Kg). As expected, TNBS administration induced a strong colitis resulting in significant body weight loss (by 16.3 ± 1.33%, Figure 3A), high macroscopic (Wallace score of 7 ± 0.25; p< 0.0001; Figure 3B) and histological (Ameho score of 6.625 ± 0.263; Figures 3C and 3D) scores of inflammation, correlated with a significant increase in the plasmatic level of IL-6 (795 pg/mL; p< 0.05; Figure 4A) and of the expression of all the pro-inflammatory genes tested (Figure 4C; p< 0.05 to 0.0001).

CNCM 1-5576 and CNCM 1-5578 were able to alleviate acute colitis.

*P. distasonis* strain CNCM 1-5578 was the most protective strain, as shown by the lowest weight loss (9.22 ± 2.14; p<0.01), reduction of the macroscopic (2.43; p< 0.0001) and histological scores (3 ± 0.65; p< 0.0001) of inflammation, leading to a protection of 65% (Figure 3A-D).

The anti-inflammatory properties of the CNCM 1-5578 strain were confirmed by a significant decrease of the plasmatic IL-6 level (67 ± 19.5 pg/mL; p<0.05) in comparison to TNBS control mice (Figure 4A) and its ability to significantly limit the expression of all the pro-inflammatory genes tested, *Il1b* (p< 0.01), *Il6* (p< 0.0001), *Tnfa* (p<0.01) and *Cxcl2* (p<0.05) (Figure 4C).

*P. distasonis* strain CNCM 1-5576 also significantly protected mice from colitis, as shown by the significant decrease in the macroscopic (3.68 ± 0.5; p<0.0001) and histological (3.5 ± 0.26; p<0.0001) score of inflammation and leading to a 47% protection. This was correlated by a significant downregulation of the expression of *Il-6, CXCL2 and Tnf-a* genes expression and to a lesser extend I1-1β (Figure 4C).

On the contrary, *P. distasonis* strain CNCM 1-5577 displayed no protective effect.

The results were corroborated with the fecal level of lipocalin 2 (Figure 4B) which was significantly elevated in TNBS control mice (p<0.05) in comparison to healthy control mice, while significantly decreased in mice treated with the strains P. distasonis CNCM 1-5578 (p<0.001) and CNCM 1-5576 (p<0.05).

As expected, no significant decrease in this pro-inflammatory marker was observed in CNCM I-5577-treated mice.

Interestingly, while TNBS treatment induced a significant drop in the expression of the tight junction protein encoding genes *Occludin* and *Zol* in comparison to healthy mice (Figure 4D; p< 0.0001 and 0.05, respectively), *P. distasonis* strain CNCM 1-5578 was able to significantly restore their expression (p< 0.05 to 0.001) while CNCM 1-5576 restored *Occludin* expression in a significant manner (p<0.05), confirming the ability of the strains to strengthen the gut barrier, as highlighted in the *in vitro* epithelial barrier model.

In contrast strain CNCM 1-5577 exhibited no ability to improve the epithelial barrier function *in vivo.*

In conclusion, the data provided by the inventors demonstrate that the two *P. distasonis* strains CNCM 1-5576 and CNCM 1-5578 exhibited the best *in vitro* anti-inflammatory profile together with a strong ability to restore the epithelial barrier and were the most potent strains in alleviating intestinal inflammation, these strains being able to significantly rescue mice from colitis.

This work allowed the inventors to determine that selected *P. distasonis* species exhibited promising potential in alleviating intestinal inflammation, but in a strain-dependent manner.

### 4. P. distasonis strains CNCM 1-5576 and CNCM 1-5578 exhibit anti-inflammatory in vitro profiles even after heat inactivation

To unravel if the anti-inflammatory potential of the strains requires viable strains, the inventors evaluated the effect of heat-killed bacteria on *in vitro* stimulation of human immune cells (PBMCs), as previously described. The two strains either alive or heat-inactivated induced very high and significant (p < 0.01 to 0.0001) levels of IL-10 and the difference between live and heat-killed bacteria was not significant (p=NS). The IL-10/IL12 ration was also similar between live and inactivated bacteria (p=NS).

On the basis of these *in vitro* results, the inventors confirmed that *P. distasonis* strains CNCM 1-5576 and CNCM 1-5578 exhibited significant inflammatory profile which was not altered after heat-inactivation, providing a rationale to use these bacteria after heat inactivation.

### 5. P. distasonis strains CNCM 1-5578 was able to limit weight gain, fat mass and local inflammation in a murine model of diet-induced obesity

The inventors evaluated *in vivo* the protective efficacy of the *P. distasonis* CNCM 1-5578 strain using a therapeutic murine model of high fat-induced obesity (HFD, 45% fat). Compared with mice fed with control low-fat diet (LFD, 10% fat), mice fed continuously with HFD expectedly showed a significant increase of body weight (Figure 6A-C), epididymal and subcutaneous adipose tissue (EWAT and SCWAT, respectively) mass and developed glucose intolerance (Figure 6 A-D). The treatment with CNCM 1-5578 performed after 8 weeks HFD diet (in obese mice) was able to limit weight gain, as shown by the reduction of body weight (in g) and weight gain (in %) either compared to the time of starting the diet or strating the bacterial treatment. CNCM 1-5578 was also able to limit the development of fat depots (visceral and sub-cutaneous, Figure 7A-B) and the local inflammation in the visceral adipose tissues, as measured by the expression of inflammatory genes (TNF-α, MCP-1) and genes corresponding to specific markers of monocyte/macrophage recruitment (F4/80, CD11b, CD11c, CD68) (Figure 8). In this model, no significant liver steatosis was observed at macroscopic level, neither inflammation of the liver (Figure 7C).

In conclusion, these data demonstrate that the *P. distasonis* strain CNCM 1-5578 exhibites a strong ability to alleviate obesity and associated inflammation.

### SEQUENCE LISTING

SEQ ID NO. 1: TGGTGTGCACAGGAGCCAAG
SEQ ID NO. 2: TTCACATCACAGCTCCCCAC
SEQ ID NO. 3: TTGACGGACCCCAAAAGATG
SEQ ID NO. 4: AGAAGGTGCTCATGTCCTCA
SEQ ID NO. 5: AGCCAGAGTCCTTCAGAGAGATAC
SEQ ID NO. 6: ATTGGATGGTCTTGGTCCTTAGC
SEQ ID NO. 7: CCCTCACACTCAGATCATCTTCTC
SEQ ID NO. 8: GGCTACAGGCTTGTCACTCG
SEQ ID NO. 9: CAAAAGATACTGAACAAAGGCAA
SEQ ID NO. 10: TCAGGTACGATCCAGGCTTCC
SEQ ID NO. 11: GACTCCAGACAACATCCCGAA
SEQ ID NO. 12: ACGCTGGAAATAACCTCGTTC
SEQ ID NO. 13: TCAGGGAATATCCACCTATCACTTCAG
SEQ ID NO. 14: CATCAGCAGCAGCCATGTACTCTTCAC
SEQ ID NO. 15: CCTGAGGGTGGGCTGGAT
SEQ ID NO. 16: GCCAATTTCCTCCGGACAT
SEQ ID NO. 17: AAACCACAGTCCCGCAGAGA
SEQ ID NO. 18: CGTGTTCACCAGCTGGCTTA
SEQ ID NO. 19: CACATCCAGCCAAAGCAGAA
SEQ ID NO. 20: GTGTCTCGGATGCTTCCACAAT
SEQ ID NO. 21: TCAGCCAGATGCAGTTAACGC
SEQ ID NO. 22: TGATCCTCTTGTAGCTCTCCAGC
SEQ ID NO. 23: TGGCGGTGGAATACAATGTG
SEQ ID NO. 24: GATGAATTCTGCGCCATGAA

Nucleic acid sequences are disclosed in the present specification that serve as references. The same sequences are also presented in a sequence listing formatted according to standard requirements for the purpose of patent matters. In case of any sequence discrepancy with the standard sequence listing, the sequences described in the present specification shall be the reference.

## Claims

1. A bacterial strain of the species *Parabacteroides distasonis* for use thereof in the treatment and/or prevention of a gastrointestinal disease, or of a disorder associated with a gastrointestinal disease, in an individual,
said bacterial strain being selected from the group consisting of:
- the bacterial strain deposited with the CNCM under accession number CNCM I-5576; and
- the bacterial strain deposited with the CNCM under accession number CNCM I-5578.

2. The bacterial strain for use thereof as claimed in claim 1, wherein the said bacterial strain is for use in the treatment and/or prevention of:
- inflammatory gastrointestinal diseases;
- disorders associated with an inflammatory gastrointestinal disease; and/or
- a leaky gastrointestinal mucosal barrier.

3. The bacterial strain for use thereof as claimed in claim 1 or 2, wherein the said bacterial strain is in a live; semi-active; inactivated, such as inactivated by heat; and/or dead form, and in particular is in a live form or in an inactivated by heat form.

4. The bacterial strain for use thereof according to any one of claims 1 to 3, **characterized in that** the individual is a mammal, in particular a human being.

5. The bacterial strain for use thereof as claimed in any one of claims 1 to 4, **characterized in that** the individual is an infant or a child, in particular is a newborn infant, and in particular is a premature infant or an infant born by caesarean section.

6. The bacterial strain for use thereof as claimed in any one of claims 2 to 5, wherein the inflammatory gastrointestinal disease is an inflammatory bowel disease.

7. The bacterial strain for use thereof as claimed in claim 6, **characterized in that** the inflammatory gastrointestinal disease is a colonic inflammatory bowel disease.

8. The bacterial strain for use thereof as claimed in claim 6 or 7, **characterized in that** the inflammatory bowel disease is chosen from the group consisting of Crohn's disease, ulcerative colitis and pouchitis, and is in particular chosen from the group consisting of Crohn's disease and ulcerative colitis.

9. The bacterial strain for use thereof as claimed in any one of claims 2 to 8, **characterized in that** the disorder associated with an inflammatory gastrointestinal disease is obesity.

10. The bacterial strain for use thereof as claimed in any one of claims 1 to 9, **characterized in that** the bacterial strain is in a composition comprising a physiologically acceptable medium.

11. The bacterial strain for use thereof as claimed in claim 10, **characterized in that** the composition comprises:
- the bacterial strain deposited with the CNCM under accession number CNCM I-5576; and
- the bacterial strain deposited with the CNCM under accession number CNCM I-5578.

12. The bacterial strain for use thereof as claimed in claim 10 or 11, wherein the composition is for oral administration and is in particular chosen from the group consisting of a food product, a beverage, a pharmaceutical product, a nutraceutical, a food additive, a food supplement and a milk product and is in particular a food supplement.

13. The bacterial strain for use according to any one of claims 10 to 12, wherein the composition further comprises at least one from the list of ingredients consisting of antioxidants, fish oils, DHA, EPA, vitamins, minerals, phytonutrients, a protein, a lipid, probiotics, prebiotics, postbiotics, polyphenols, flavonoids, and combinations thereof.

14. A bacterial strain deposited with the CNCM under accession number CNCM I-5576.

15. A bacterial strain deposited with the CNCM under accession number CNCM I-5578.

16. A composition comprising, in a physiologically acceptable medium, at least a bacterial strain as defined in claim 14 and/or in claim 15.


SEQUENCE LISTING

<110> Institut national de recherche pour lagriculture, lalimentation et
lenvironnement
      Université de Paris
      Institut Pasteur de Lille
      Institut national de la santé et de la recherche médicale
      Centre National de la Recherche Scientifique
      Université de Lille

<120> PARABACTEROIDES DISTASONIS STRAINS FOR USE THEREOF IN THE TREATMENT
AND PREVENTION OF GASTROINTESTINAL DISEASES AND OF DISORDERS ASSOCIATED WITH
GASTROINTESTINAL DISEASES

<130> PR87528/MGG

<160> 24

<170> BiSSAP 1.3.6

<210> 1
<211> 20
<212> DNA
<213> Artificial Sequence

<220>
<223> Tbp-F

<400> 1
tggtgtgcac aggagccaag                                                   20

<210> 2
<211> 20
<212> DNA
<213> Artificial Sequence

<220>
<223> Tbp-R

<400> 2
ttcacatcac agctccccac                                                   20

<210> 3
<211> 20
<212> DNA
<213> Artificial Sequence

<220>
<223> Il1b-F

<400> 3
ttgacggacc ccaaaagatg                                                   20

<210> 4
<211> 20
<212> DNA
<213> Artificial Sequence

<220>
<223> Il1b-R

<400> 4
agaaggtgct catgtcctca                                                    20


<210> 5
<211> 24
<212> DNA
<213> Artificial Sequence


<220>
<223> il6-F

<400> 5
agccagagtc cttcagagag atac                                               24


<210> 6
<211> 23
<212> DNA
<213> Artificial Sequence


<220>
<223> il6-R

<400> 6
attggatggt cttggtcctt agc                                                23


<210> 7
<211> 24
<212> DNA
<213> Artificial Sequence


<220>
<223> tnfa-F

<400> 7
ccctcacact cagatcatct tctc                                               24


<210> 8
<211> 20
<212> DNA
<213> Artificial Sequence


<220>
<223> tnfa-R

<400> 8
ggctacaggc ttgtcactcg                                                    20


<210> 9
<211> 23

<212> DNA
<213> Artificial Sequence

<220>
<223> cxcl2-f

<400> 9
caaaagatac tgaacaaagg caa                                              23

<210> 10
<211> 21
<212> DNA
<213> Artificial Sequence

<220>
<223> cxcl2-R

<400> 10
tcaggtacga tccaggcttc c                                                21

<210> 11
<211> 21
<212> DNA
<213> Artificial Sequence

<220>
<223> zo1-F

<400> 11
gactccagac aacatcccga a                                                21

<210> 12
<211> 21
<212> DNA
<213> Artificial Sequence

<220>
<223> zo1-R

<400> 12
acgctggaaa taacctcgtt c                                                21

<210> 13
<211> 27
<212> DNA
<213> Artificial Sequence

<220>
<223> occludin-F

<400> 13
tcagggaata tccacctatc acttcag                                          27

<210> 14
<211> 27
<212> DNA
<213> Artificial Sequence

<220>
<223> occludin-R

<400> 14
catcagcagc agccatgtac tcttcac                                          27

<210> 15
<211> 18
<212> DNA
<213> Artificial Sequence

<220>
<223> CD11c-F

<400> 15
cctgagggtg ggctggat                                                    18

<210> 16
<211> 19
<212> DNA
<213> Artificial Sequence

<220>
<223> CD11c-R

<400> 16
gccaatttcc tccggacat                                                   19

<210> 17
<211> 20
<212> DNA
<213> Artificial Sequence

<220>
<223> CD11b-F

<400> 17
aaaccacagt cccgcagaga                                                  20

<210> 18
<211> 20
<212> DNA
<213> Artificial Sequence

<220>
<223> CD11b-R

<400> 18
cgtgttcacc agctggctta                                                  20

<210> 19
<211> 20
<212> DNA
<213> Artificial Sequence

<220>
<223> F4/80-F

<400> 19
cacatccagc caaagcagaa                                                    20

<210> 20
<211> 22
<212> DNA
<213> Artificial Sequence

<220>
<223> F4/80-R

<400> 20
gtgtctcgga tgcttccaca at                                                 22

<210> 21
<211> 21
<212> DNA
<213> Artificial Sequence

<220>
<223> MCP1-F

<400> 21
tcagccagat gcagttaacg c                                                  21

<210> 22
<211> 23
<212> DNA
<213> Artificial Sequence

<220>
<223> MCP1-R

<400> 22
tgatcctctt gtagctctcc agc                                                23

<210> 23
<211> 20
<212> DNA
<213> Artificial Sequence

<220>
<223> CD68-F

<400> 23
tggcggtgga atacaatgtg                                                    20

<210> 24
<211> 20
<212> DNA
<213> Artificial Sequence

<220>
<223> CD68-R

<400> 24
gatgaattct gcgccatgaa                                                    20